(19) Europäisches Patentamt
European Patent Office
Office européen des brevets

(11) **EP 2 150 515 B1**

(12) **EUROPÄISCHE PATENTSCHRIFT**


(45) Veröffentlichungstag und Bekanntmachung des Hinweises auf die Patenterteilung:
**24.08.2016 Patentblatt 2016/34**

(21) Anmeldenummer: **08736561.5**

(22) Anmeldetag: **24.04.2008**

(51) Int Cl.:
***C07C 43/11*** *(2006.01)* ***A01N 25/30*** *(2006.01)*
***A01N 43/653*** *(2006.01)* ***C08G 65/26*** *(2006.01)*

(86) Internationale Anmeldenummer:
**PCT/EP2008/055034**

(87) Internationale Veröffentlichungsnummer:
**WO 2008/132150 (06.11.2008 Gazette 2008/45)**


(54) **ALKOHOLALKOXYLATE, DIESE ENTHALTENDE MITTEL UND VERWENDUNG DER ALKOHOLALKOXYLATE ALS ADJUVANS FÜR DEN AGROCHEMISCHEN BEREICH**

ALCOHOL ALKOXYLATES, AGENTS CONTAINING THESE, AND USE OF THE ALCOHOL ALKOXYLATES AS ADJUVANTS FOR THE AGROCHEMICAL FIELD

ALCOXYLATES D'ALCOOL, PRODUITS CONTENANT CES COMPOSES ET UTILISATION DE CES ALCOXYLATES D'ALCOOL COMME ADJUVANTS DANS LE DOMAINE AGROCHIMIQUE


(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **25.04.2007 EP 07106961**

(43) Veröffentlichungstag der Anmeldung:
**10.02.2010 Patentblatt 2010/06**



(73) Patentinhaber: **BASF SE**
**67056 Ludwigshafen am Rhein (DE)**

(72) Erfinder:
- **STÖSSER, Michael**
  **67141 Neuhofen (DE)**
- **BERGHAUS, Rainer**
  **67346 Speyer (DE)**
- **KRENNRICH, Gerhard**
  **67227 Frankenthal (DE)**
- **KUMMETER, Markus**
  **68542 Heddesheim (DE)**
- **OETTER, Günter**
  **67227 Frankenthal (DE)**
- **STEINBRENNER, Ulrich**
  **67435 Neustadt (DE)**
- **WAGNER, Norbert**
  **67112 Mutterstadt (DE)**
- **DOMBO, Peter**
  **65207 Wiesbaden (DE)**

(74) Vertreter: **BASF IP Association**
**BASF SE**
**ZRX-C6**
**67056 Ludwigshafen (DE)**



(56) Entgegenhaltungen:
**EP-A2- 0 261 492** **WO-A-99/03345**
**WO-A-03/090531** **WO-A-2005/084435**
**GB-A- 2 109 403** **JP-A- 6 313 191**
**US-A- 6 025 318** **US-A- 6 071 857**

- **DATABASE WPI Week 199013 Thomson Scientific, London, GB; AN 1990-096542 XP002455049 & JP 02 049701 A (TAKEMOTO OIL & FAT CO LTD) 20. Februar 1990 (1990-02-20)**
- **DATABASE WPI Week 200167 Thomson Scientific, London, GB; AN 2001-592518 XP002455050 & JP 2001 163813 A (LION CORP) 19. Juni 2001 (2001-06-19)**
- **DATABASE WPI Week 199715 Thomson Scientific, London, GB; AN 1997-161445 XP002455051 & JP 09 031004 A (SANYO CHEM IND LTD) 4. Februar 1997 (1997-02-04)**
- **DATABASE WPI Week 200653 Thomson Scientific, London, GB; AN 2006-521936 XP002455052 & WO 2006/070816 A1 (MITSUBISHI ELECTRIC CORP) 6. Juli 2006 (2006-07-06)**
- **DATABASE BEILSTEIN BEILSTEIN INSTITUTE FOR ORGANIC CHEMISTRY, FRANKFURT-MAIN, DE; XP002455025 Database accession no. BRN 6797224 & E. A. EL'PERINA ET AL.: RUSS. CHEM. Bl., Bd. 42, Nr. 4, 1993, Seiten 744-750,**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- DATABASE BEILSTEIN BEILSTEIN INSTITUTE FOR ORGANIC CHEMISTRY, FRANKFURT-MAIN, DE; XP002455026 Database accession no. BRN 1920968 & MOVSUMZADE ET AL.: DOKL. AKAD.NAUK AZ. SSR, Bd. 33, Nr. 7, 1977, Seite 48, 49, 50, 51,
- DATABASE BEILSTEIN BEILSTEIN INSTITUTE FOR ORGANIC CHEMISTRY, FRANKFURT-MAIN, DE; XP002455027 Database accession no. BRN 6796816, 6964705 & JANIK, RYSZARD: POL. J. CHEM., Bd. 60, Nr. 4-6, 1986, Seiten 593-598,

## Beschreibung

Die vorliegende Erfindung betrifft bestimmte Alkoholalkoxylate vom amphiphilen Typ, agrochemische Mittel, die diese enthalten, und die Verwendung der Alkoholalkoxylate als wirkungsverbesserndes Adjuvans im agrochemischen Bereich und insbesondere im Bereich des Pflanzenschutzes.

Neben der Optimierung der Wirkstoffeigenschaften kommt mit Blick auf eine industrielle Produktion und Anwendung von Wirkstoffen der Entwicklung eines effizienten Mittels besondere Bedeutung zu. Durch eine sachgerechte Formulierung des oder der Wirkstoffe muß ein optimaler Ausgleich zwischen teils gegenläufigen Eigenschaften wie der biologischen Wirksamkeit, der Toxikologie, möglichen Einflüssen auf die Umwelt und den Kosten gefunden werden. Darüber hinaus bestimmt die Formulierung zu einem erheblichen Maß die Haltbarkeit und den Anwendungskomfort eines Mittels.

Von besonderer Bedeutung für die Wirksamkeit eines agrochemischen Mittels ist die effektive Aufnahme des Wirkstoffs durch die Pflanze. Erfolgt diese Aufnahme über das Blatt, so stellt sich diese als komplexer Transportvorgang dar, bei dem die Wirkstoffmasse, beispielsweise ein Herbizid, zunächst in die wachsartige Cuticula des Blatts eindringen und anschließend über die Cuticula in die unterliegenden Gewebe an den eigentlichen Wirkort diffundieren muss.

Allgemein bekannt und landwirtschaftliche Praxis ist es, zwecks verbesserter Wirksamkeit Formulierungen bestimmte Hilfsstoffe zuzusetzen. Vorteilhafterweise können dadurch die Wirkstoffmengen in der Formulierung bei gleichbleibender Aktivität verringert werden, wodurch Kosten minimiert und gegebenenfalls bestehende gesetzliche Regelungen eingehalten werden können. Auch gelingt es in Einzelfällen, das Wirkstoffspektrum zu vergrößern, indem Pflanzen, die ohne Zusatz nur in unzureichender Weise mit einem bestimmten Wirkstoff behandelt werden konnten, durch Zusatz bestimmter Hilfsstoffe einer entsprechenden Behandlung zugänglich sind. Weiterhin kann die Leistungsfähigkeit unter ungünstigen Umweltbedingungen in Einzelfällen durch eine geeignete Formulierung erhöht werden. Mithin können auch Unverträglichkeiten verschiedener Wirkstoffe in einer Formulierung vermieden werden.

Derartige Hilfsstoffe werden gelegentlich auch als Adjuvantien bezeichnet. Es handelt sich oftmals um oberflächenaktive oder salzartige Verbindungen. Je nach Wirkungsweise können z.B. Modifikatoren, Aktuatoren, Dünger und pH-Puffer unterschieden werden. Modifikatoren beeinflussen Benetzung, Haftung und Spreitung einer Formulierung. Aktuatoren brechen die wachsartige Pflanzencuticula auf und verbessern die Penetration des Wirkstoffs in die Cuticula sowohl kurzfristig (im Minutenbereich) als auch langfristig (im Stundenbereich). Dünger wie Ammoniumsulfat, Ammoniumnitrat oder Harnstoff verbessern die Absorption und Löslichkeit des Wirkstoffs, und sie können antagonistische Verhaltensweisen von Wirkstoffen verringern. pH-Puffer werden herkömmlicherweise zur optimalen Einstellung des pH-Werts der Formulierung verwendet.

Im Hinblick auf die Aufnahme des Wirkstoffs in das Blatt können oberflächenaktive Substanzen als Modifikatoren und Aktuatoren wirken. Allgemein wird angenommen, dass geeignete oberflächenaktive Substanzen die effektive Kontaktfläche von Flüssigkeiten auf Blättern durch eine Verminderung der Oberflächenspannung erhöhen können. Darüber hinaus können bestimmte oberflächenaktive Substanzen die epicuticulären Wachse auflösen oder aufbrechen, was die Absorption des Wirkstoffs erleichtert. Ferner können einige oberflächenaktive Substanzen auch die Löslichkeit von Wirkstoffen in Formulierungen verbessern und damit eine Kristallbildung vermeiden oder diese zumindest hinauszögern. Schließlich können sie in bestimmten Fällen auch die Absorption von Wirkstoffen beeinflussen, indem sie Feuchtigkeit zurückhalten.

Adjuvantien vom oberflächenaktiven Typ werden in vielfältiger Weise für agrochemische Anwendungen genutzt. Man kann diese in anionische, kationische, nicht-ionische oder amphotere Stoffgruppen unterteilen.

Traditionell werden Öle auf Petroleum-Basis als aktivierende Adjuvantien verwendet. In jüngster Vergangenheit setzte man auch Samenextrakte, natürliche Öle und deren Derivate, beispielsweise aus Sojabohnen, Sonnenblumen und Kokosnuss, ein.

Bei synthetischen oberflächenaktiven Substanzen, die üblicherweise als Aktuatoren verwendet werden, handelt es sich unter anderem um Polyoxyethylen-Kondensate mit Alkoholen, Alkylphenolen oder Alkylaminen, welche HLB-Werte im Bereich von 8 bis 13 aufweisen. In diesem Sinne nennt die WO 00/42847 beispielsweise den Einsatz bestimmter linearer Alkoholalkoxylate, um die Wirksamkeit agrochemischer Biozidformulierungen zu steigern.

Das Spektrum von Alkoholalkoxylaten ist jedoch vielfältig. Als Tenside finden sie vornehmlich Anwendung in Wasch- und Reinigungsmitteln, in der metallverarbeitenden Industrie, bei der Herstellung und Verarbeitung von Textilien, in der Leder-, Papier-, Druck-, Galvano- und Fotoindustrie, bei der Wasserbehandlung, in pharmazeutischen, tierarzneilichen und den Pflanzenschutz betreffenden Formulierungen, oder in der Kunststoff herstellenden und Kunststoff verarbeitenden Industrie. Insbesondere die Strukturen des Alkoholteils und in bestimmten Fällen auch des Alkoxylatteils nehmen Einfluss auf die Eigenschaften der Alkoxylate, so dass bei den vorstehend genannten Anwendungen verschiedene technische Effekte zum Tragen kommen können. Hierzu gehören das Benetzen, das Spreiten, die Penetration, das Anhaften, die Filmbildung, das Verbessern von Verträglichkeiten, die Driftkontrolle und das Entschäumen.

WO 03/090531 beschreibt die Verwendung von Alkoxylaten bestimmter verzweigter Alkohole, zu denen insbesondere 2-Propylheptanol, C13-Oxoalkohole und C10-Oxoalkohole gehören, als Adjuvans für den agrochemischen

Bereich. Ähnliche Alkohololkoxylate werden in der WO 2005/015998 speziell als Adjuvans für Fungizide Benzamidoxim-Derivate vorgeschlagen. WO 00/35278 betrifft agrochemische Formulierungen auf Basis von PO/EO-Blockcopolymeren des 2-Ethylhexanols. Die in WO 99/03345 als Adjuvans zur Verbesserung der Wirksamkeit eines Pestizids und Pflanzenschutzmittels beschriebenen EO/PO-EO-Alkoxylate basieren auf Alkoholen mit mehr als 14 Kohlenstoffatomen. WO 2005/084435 beschreibt Suspensionskonzentrate auf Ölbasis, die eines der beiden endgruppenverschlossenen Alkoholblockalkoxylate $CH_3$-$(CH_2)_{10}$-O-$(EO)_6$-$(BO)_2$-$CH_3$ oder $CH_3$-$(CH_2)_8$-O-$(EO)_8$-$(BO)_2$-$CH_3$ als Penetrationsförderer enthalten. Diese endruppenverschlossenen Alkoholblockalkoxylate sollen eine bessere Wirkung besitzen als vergleichbare Alkoxylate ohne Endgruppenverschluss.

Sämtliche jener Alkoholalkoxylate basieren also auf langkettigen Alkoholen mit wenigstens 8 Kohlenstoffatomen auf. Es sind aber auch vereinzelt Alkoholalkoxylate auf Basis kurzkettiger Alkohole bekannt.

So sind beispielsweise in US 6,071,857 flüssige Pestizidzusammensetzungen beschrieben, die das unter dem Handelsnamen Witconol NS 500 K® bekannte oberflächenaktive Butanol-EO/PO-Blockalkoxylat enthalten.

JP 02049701 beschreibt Pflanzenschutzgranulate, die als Zerfallshilfsmittel EO/POalkoxylate des n-Butanols enthalten.

EP 0 261 492 beschreibt Suspoemulsionen, die ein oberflächenaktives Ethylenoxid-Propylenoxid-Blockpolymer mit einem relativ hohen mittleren Molekulargewicht zur Stabilisierung der Suspoemulsion enthalten.

JP 06313191 beschreibt die Anwendung der Alkoholalkoxylate $C_4H_9$-O-$(C_3H_6O)_2$-$(C_2H_4O)_8$-$(C_3H_6O)_2$-H, $C_4H_9$-O-$(C_3H_6O)_4$-$(C_2H_4O)_{12}$-$(C_3H6_O)_1$-H und $C_6H_{13}$-O-$(C_3H_6O)_2$-$(C_2H_4O)_{12}$-$(C_3H_6O)_1$-H in Wasch- und Reinigungsmitteln.

GB 2 109 403 beschreibt die Anwendung eines Mono-$C_1C_6$-Alkylethers von Ethoxylat/Propoxylat-Copolymeren mit einem Ethoxylat:Propoxylat-Gewichtsverhältnis im Bereich von 30:70 bis 5:95 in Textilveredelungsmitteln.

JP 2001 163813 beschreibt Alkoholalkoxylate vom Typ $C_4$-$C_8$-Alkyl-O-$(C_2H_4O)_{1-2}$-$(C_3H_6O)_{0.1-1}$-$C_2H_4O)_{1-5}$-H und deren Verwendung als Lösungsmittel oder Wärmeträger.

JP 09 031004 scheint EO/PO-Alkoxylate von Heptanolen und deren Verwendung als Lösungsmittel mit oberflächenaktiver und emulgierender Wirkung zu bechreiben.

WO 2006/070816 scheint EO/PO-Alkoxylate von n-Propanol und deren Verwendung als Tensid zur Bildung eines stabilen Schaums zu bechreiben.

US 6,025,318 beschreibt Wasch- und Reinigungsmitteln, die EO/PO-Alkoxylate von iso-C13-Alkoholen und Hexanol enthalten.

Allerdings zieht keine dieser Druckschriften die Anwendung der Alkoholalkoxylate auf Basis kurzkettiger Alkohole als wirkungssteigerndes Adjuvans im Pflanzenschutzbereich in Erwägung.

Der vorliegenden Erfindung liegt die Aufgabe zugrunde, weitere für den agrochemischen Bereich brauchbare Adjuvantien zur Verfügung zu stellen.

Diese Aufgabe löst die vorliegende Erfindung mit bestimmten Alkoxylaten kurzkettiger Alkohole, zu denen vor allem PO/EO-, EO/PO- und EO/PO/EO-Blockalkoxylate bzw. BO/EO-, EO/BO- und EO/BO/EO-Blockalkoxylate gehören, deren Verwendung als Adjuvans und agrochemischen Mitteln, die diese Alkoxylate enthalten.

Gegenstand der vorliegenden Erfindung sind Mittel, umfassend

(a) wenigstens einen Wirkstoff zur Pflanzenbehanldung: und
(b) wenigstens einen alkoxylierten Alkohol der Formel (IIa)

$$R\text{-}O\text{-}(C_mH_{2m}O)_q\text{-}(C_nH_{2n}O)_x\text{-}(C_2H_4O)_y\text{-}Z \qquad \text{(IIa)}$$

worin

| | |
|---|---|
| R | für $C_1C_7$-Alkyl steht; |
| m | für 2 oder 3 steht; |
| q | für 0, 1, 2 oder 3 steht; |
| n | für eine ganze Zahl von 3 bis 16 steht; |
| x | für einen Wert von 1 bis 100 steht; |
| y | größer als 0 ist und für einen Wert bis 100 steht; |
| x+y | einem Wert von 10 bis 30 entspricht; und |
| Z | für Wasserstoff oder $C_1$-$C_3$-Alkyl steht, |

wobei das Gewichtsverhältnis von Komponente (b) zu Komponente (a) größer als 1 ist.

Die in den erfindungsgemäßen Mitteln enthaltenen Alkoholalkoxylate weisen vor allem adjuvante, insbesondere wirkungsfördernde Eigenschaften auf. So ermöglicht der Zusatz derartiger Alkoxylate eine beschleunigte Aufnahme von Wirkstoffen durch eine mit dem Wirkstoff zu behandelnde Pflanze. Aus der adjuvanten Wirkung leiten sich insbesondere

folgende Aspekte bei der Behandlung von Pflanzen mit einem oder mehreren Wirkstoffen ab:

- vergleichsweise höhere Wirksamkeit des Wirkstoffs bei gegebener Aufwandmenge;
- vergleichsweise geringere Aufwandmenge bei gegebener Wirkung;
- vergleichsweise stärkere Aufnahme des Wirkstoffs durch die Pflanze, insbesondere über das Blatt, und damit Vorteile im Nachlaufverfahren, insbesondere bei der Sprühbehandlung von Pflanzen.

Demnach betrifft die vorliegende Erfindung auch Verfahren zur Behandlung von Pflanzen, wobei man eine geeignete Menge an erfindungsgemäßem Mittel appliziert.

Besondere Vorteile werden insbesondere im Anbau von Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napo-brassica, Brassica rapa var. silvestris, Brassica aleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Ribes sylvestre, Ricinus communis, Saccharum officinarum, Secale cereale, Sinapsis alba, Solanum tuberosum, Sorghum bicolor (s. vulgare), Theobroma cacao, Trifolium pratense, Triticale, Triticum aestivum, Triticum durum, Vicia faba, Vitis vinifera, Zea mays erzielt.

Besondere Wirkungen erzielen die erfindungsgemäßen Mitte! im Anbau von Allium cepa, Hordeum vulgare, Triticum aestivum und Triticum durum.

Darüber hinaus können die erfindungsgemäß zu verwendenden Mittel auch in Kulturen, die gegen die Wirkung von Pestiziden, insbesondere Herbiziden, tolerant sind, verwendet werden. Derartige Kulturen können beispielsweise durch Züchtung und auch gentechnische Methoden erhalten werden.

Der Alkoholteil der erfindungsgemäßen Alkoholalkoxylate basiert in der Regel auf an sich bekannten Alkoholen oder Alkoholgemischen mit 1 bis 7 und insbesondere mit entweder 1 bis 3, 4, oder 5 bis 7, also 5, 6 oder 7 Kohlenstoffatomen. Somit steht R in Formel (I) insbesondere für Methyl, Ethyl, n-Propyl, iso-Propyl, n-Butyl, 2-Butyl, isoButyl or tert-Butyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, 2-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 3-Ethylbutyl, 1,1,2-Trimethylpropyl, 1,2,2-Trimethylpropyl, 1-Ethyl-1-methylpropyl,1-Ethyl-2-methylpropyl, 2-Ethyl-1-methylpropyl, 2-Ethyl-2-methylpropyl, n-Heptyl, 1-Methylhexyl, 2-Methylhexyl, 3-Methylhexyl, 4-Methylhexyl, 5-Methylhexyl, 1,1-Dimethylpentyl, 1,2-Dimethylpentyl, 1,3-Dimethylpentyl, 1,4-Dimethylpentyl, 2,2-Dimethylpentyl, 2,3-Dimethylpentyl, 2,4-Dimethylpentyl, 3,3-Dimethylpentyl, 3,4-Dimethylpentyl, 4,4-Dimethylpentyl, 1-Ethylpentyl, 2-Ethylpentyl, 3-Ethylpentyl, 4-Ethylpentyl, 1,1,2-Trimethylbutyl, 1,1,3-Trimethylbutyl, 1,2,2-Trimethylbutyl, 1,2,3-Trimethylbutyl, 1,3,3-Trimethylbutyl, 1-Ethyl-1-methylbutyl,1-Ethyl-2-methylbutyl, 1-Ethyl-3-methylbutyl, 2-Ethyl-1-methylbutyl, 2-Ethyl-2-methylbutyl, 2-Ethyl-3-methylbutyl, 3-Ethyl-1-methylbutyl, 3-Ethyl-2-methylbutyl, 3-Ethyl-3-methylbutyl, 1-Propylbutyl, 2-Propylbutyl, 3-Propylbutyl, 1-Butylpropyl, 2-Butylpropyl, 1-Propyl-1-methylpropyl, 1-Propyl-2-methylpropyl, 2-Propyl-1-methylpropyl, 2-Propyl-2-methylpropyl, 1,1-Diethylpropyl, 1,2-Diethylpropyl, oder 2,1-Diethylpropyl, wobei auch Gemische aus zwei oder mehr Alkoholalkoxylaten, in denen R verschieden ist, geeignet sein können.

Der Alkoholteil der Alkoxylate ist daher linear oder verzweigt. Zu den linearen Alkoholen gehören insbesondere Methanol, Ethanol, n-Propanol, n-Butanol, n-Pentanol und n-Hexanol, außerdem n-Heptanol. Von den verzweigten ist insbesondere die verzeigten Pentanole (Amylalkohole) zu nennen, die häufig als Gemisch eingesetzt werden. Solche mit 5, 6 oder 7 Kohlenstoffatomen haben sich als besonderes vorteilhaft herausgestellt.

Gemäß einer besonderen Ausführungsform ist der alkoxylierte Alkohol ausgewählt unter alkoxylierten Alkoholen der Formel (I), worin q für 1, 2 oder 3 steht. Solche Alkoholalkoxylate lassen sich gezielt herstellen, indem man beispielsweise eine Verbindung der Formel (XI)

$$R\text{-}O\text{-}(C_mH_{2m}O)_q\text{-}H \qquad (XI)$$

worin

R, m und q wie hierin definiert sind;
in der oben beschriebenen Weise alkoxyliert und gegebenenfalls endgruppenverschließt.

Bei den Verbindungen der Formel (XI) handelt es sich um an sich bekannte Mono-$C_1$-$C_7$-Alkylether des Ethy-

lenglykols (m = 2; q = 1), Propylenglykols (m = 3; q = 1), Diethylenglykols (m = 2; q = 2), Dipropylenglykols (m = 3; q = 2), Triethylenglykols (m = 2; q = 3) oder Tripropylenglykols (m = 3; q = 3).

Erfindungsgemäß von besonderer Bedeutung sind die Alkylether des Mono-, Di- und Tripropylenglykols der Formel (XIa)

$$R\text{-}O\text{-}(CH_2CH(CH_3)O)_q\text{-}H \qquad \text{(XIa)}$$

worin R und q wie hierin definiert ist.

Einem anderen Aspekt zufolge sind die Mono-$C_1$-$C_4$-Alkylether (d.h. R steht insbesondere für $C_1$-$C_4$-Alkyl) und hiervon vor allem die Dipropylenglykolmonoalkylether (q = 2) hervorzuheben. Hierzu gehören insbesondere Dipropylenglykolmonomethylether, Dipropylenglykolmonoethylether, Dipropylenglykolmono-n-propylether und Dipropylenglykolmono-n-butylether.

Geeignete Alkohole sind sowohl aus nativen Quellen als auch auf synthetischem Weg, z. B. durch Aufbau aus Edukten mit einer geringeren Zahl an Kohlenstoffatomen erhältlich.

Die Alkoxylierung ergibt sich aus der Umsetzung mit geeigneten Alkylenoxiden, nämlich mit Ethylenoxid (EO) und wenigstens einem höheren, 3 bis 16 und vorzugsweise 3 bis 6 Kohlenstoffatome aufweisenden Alkylenoxid, von denen insbesondere 1,2-Propylenoxid (PO), 1,2-Butylenoxid (BO), 1,2-Pentylenoxid (PeO) und 1,2-Hexylenoxid (HO) zu nennen sind. Des Weiteren ist auch 1,2-Decylenxid (DeO) zu nennen.

In Abhängigkeit von den für die Umsetzung gewählten Einsatzmengen an Alkylenoxid(en) sowie den Reaktionsbedingungen ergibt sich der jeweilige Alkoxylierungsgrad. Hierbei handelt es sich in der Regel um einen statistischen Mittelwert, da die Anzahl von Alkylen-oxid-Einheiten der aus der Umsetzung resultierenden Alkoholalkoxylate variiert.

Der Alkoxylierungsgrad, d.h. die mittlere Kettenlänge der Polyetherketten der in den erfindungsgemäßen Mitteln enthaltenen Alkoholalkoxylate und deren Zusammensetzung (sprich die Werte von x, und y) kann durch das bei Ihrer Herstellung eingesetzte Molmengenverhältnis von Alkohol zu Ethylenoxid und höherem Alkylenoxid sowie den Reaktionsbedingungen gesteuert werden. Einerseits umfassen die erfindungsgemäßen Alkoholalkoxylate bevorzugt mindestens oder mehr als 2, bevorzugter mindestens oder mehr als 4, insbesondere mindestens oder mehr als 6, vor allem mindestens oder mehr als 8, und besonders bevorzugt mindestens oder mehr als 10 Alkylenoxid-Einheiten. Andererseits umfassen die erfindungsgemäßen Alkoholalkoxylate bevorzugt höchstens oder weniger als 100, 90 oder 85, bevorzugter höchstens oder weniger als 80, 70 oder 65, insbesondere höchstens oder weniger als 60, 50 oder 45, vor allem höchstens oder weniger als 40 oder 35, und besonders bevorzugt höchstens oder weniger als 30 oder 25 Alkylenoxid-Einheiten. Bevorzugt sind demnach beispielsweise Alkoholalkoxylate mit 2 bis 100, 90 oder 85, wie 4 bis 80, 70 oder 65, insbesondere 6 bis 60, 50 oder 45, vor allem 8 bis 40 oder 35, und besonders 10 bis 30 oder 25 Alkylenoxid-Einheiten (x+y).

Gemäß einer besonderen Ausführungsform betrifft die Erfindung Mittel, umfassend Alkoholalkoxylate der Formel (I), worin der Wert von x größer als der Wert von y ist. Hierbei handelt es sich um Alkoholalkoxylate, die mehr Einheiten des höheren Alkylenoxids als Ethylenoxid-Einheiten aufweisen. Dies gilt vor allem für Alkoholalkoxylate der Formel (I), worin n 3 ist.

Einerseits weisen solche Alkoholalkoxylate einen Alkoxylatteil auf, im dem das Verhältnis von höherem Alkylenoxid zu Ethylenoxid- (x zu y) wenigstens oder mehr als 1,1:1, vorzugsweise wenigstens oder mehr als 1,5:1, vor allem wenigstens oder mehr als 1,8:1, und insbesondere wenigstens oder mehr als 2:1, beispielsweise wenigstens oder mehr als 3:1, beträgt. Andererseits weisen solche Alkoholalkoxylate einen Alkoxylatteil auf, in dem das Verhältnis von höherem Alkylenoxid zu Ethylenoxid (x zu y) höchstens oder weniger als 25:1, vorzugsweise höchstens oder weniger als 15:1, und insbesondere höchstens oder weniger als 10:1, beispielsweise höchstens oder weniger als 4,3:1, beträgt. Bevorzugt sind demnach Alkoxylate, bei denen das Verhältnis von höherem Alkylenoxid zu Ethylenoxid (x zu y) 1,1:1 bis 25:1, vorzugsweise 1,5:1 bis 15:1, vor allem 1,8 bis 15:1, und insbesondere 2:1 bis 10:1, beispielsweise 2,3:1 bis 4,3:1, beträgt. Dies gilt vor allem für Alkoholalkoxylate der Formel (I), worin n 3 oder 4 ist.

Gemäß einer weiteren besonderen Ausführungsform betrifft die Erfindung Mittel, umfassend Alkoholalkoxylate der Formel (I), worin der Wert von x kleiner als der Wert von y ist. Hierbei handelt es sich um Alkoholalkoxylate, die weniger Einheiten des höheren Alkylenoxids als Ethylenoxid-Einheiten aufweisen. Dies gilt vor allem für Alkoholalkoxylate der Formel (I), worin n größer als 3, also 4, oder vor allem, 5, 6 oder 10 ist.

Einerseits weisen solche Alkoholalkoxylate einen Alkoxylatteil auf, im dem das Verhältnis von Ethylenoxid zu höherem Alkylenoxid (y zu x) wenigstens oder mehr als 1,1:1, vorzugsweise wenigstens oder mehr als 1,5:1, vor allem wenigstens oder mehr als 2:1, und insbesondere wenigstens oder mehr als 2,5:1, beispielsweise wenigstens oder mehr als 3:1, beträgt. Andererseits weisen solche Alkoholalkoxylate einen Alkoxylatteil auf, im dem das Verhältnis von Ethylenoxid zu höherem Alkylenoxid (y zu x) höchstens oder weniger als 25:1, vorzugsweise höchstens oder weniger als 20:1, und insbesondere höchstens oder weniger als 15:1, beispielsweise höchstens oder weniger als 10:1, beträgt. Bevorzugt sind demnach Alkoxylate, bei denen das Verhältnis von Ethylenoxid zu höherem Alkylenoxid (y zu x) 1,1:1 bis 25:1, vorzugsweise 1,5:1 bis 20:1 und insbesondere 2:1 bis 15:1, beispielsweise 2,5:1 bis 10:1, beträgt. Dies gilt vor allem für Alkoholalkoxylate der Formel (I), worin n größer als 3, also vor allem 5, 6 oder 10 ist.

Der den höheren Alkylenoxid-Einheiten zuzurechnende Alkoxylierungsgrad (Wert von x) beträgt einerseits in der Regel 1 bis 50, vorzugsweise 3 bis 30 und insbesondere 5 bis 20. Dies gilt vor allem für Alkoholalkoxylate der Formel (I), worin n 3 oder 4 ist.

Der den höheren Alkylenoxid-Einheiten zuzurechnende Alkoxylierungsgrad (Wert von x) beträgt andererseits in der Regel 1 bis 30, vorzugsweise 2 bis 20 und insbesondere 3 bis 15. Dies gilt vor allem für Alkoholalkoxylate der Formel (I), worin n größer als 3, also 4, oder vor allem 5, 6 oder 10 ist.

Der den Ethylenoxid-Einheiten zuzurechnende Alkoxylierungsgrad (Wert von y) beträgt einerseits in der Regel 1 bis 30, vorzugsweise 2 bis 20 und insbesondere 3 bis 10. Dies gilt vor allem für Alkoholalkoxylate der Formel (I), worin n 3 oder 4 ist.

Der den Ethylenoxid-Einheiten zuzurechnende Alkoxylierungsgrad (Wert von y) beträgt andererseits in der Regel 1 bis 50, vorzugsweise 3 bis 40 und insbesondere 5 bis 30. Dies gilt vor allem für Alkoholalkoxylate der Formel (I), worin n größer als 3, also 4, oder vor allem 5, 6 oder 10 ist.

Die Umsetzung der Alkohole bzw. Alkoholgemische mit dem/den Alkylenoxid(en) erfolgt nach üblichen, dem Fachmann bekannten Verfahren und in dafür üblichen Apparaturen.

Die Alkoxylierung kann durch starke Basen, wie Alkalihydroxide und Erdalkalihydroxide, Brönstedsäuren oder Lewissäuren, wie $AlCl_3$, $BF_3$ etc. katalysiert werden. Für eng verteilte Alkoholoxylate können Katalysatoren wie Hydrotalcit oder DMC verwendet werden.

Die Alkoxylierung erfolgt vorzugsweise bei Temperaturen im Bereich von 80 bis 250 °C, bevorzugt 100 bis 220 °C. Der Druck liegt vorzugsweise zwischen Umgebungsdruck und 600 bar. Gewünschtenfalls kann das Alkylenoxid eine Inertgasbeimischung, z. B. von 5 bis 60 %, enthalten.

$-C_nH_{2n}O-$ (bei n gleich oder größer 3) bedeutet entweder $-CH(C_{n-2}H_{2n-3})CH_2O-$ (z.B.$-CH(CH_3)CH_2O-$) oder $-CH_2CH(C_{n-2}H_{2n-3})O-$ (z.B. $-CH_2CH(CH_3)O-$). Dabei kann ein bestimmtes Alkoholalkoxylat im Wesentlichen Alkylenoxid-Einheiten der einen oder der anderen Art, oder beide enthalten. Ein Alkylenoxid-Block $-(C_nH_{2n}O)_x-$ kann sich im Wesentlichen aus Alkylenoxid-Einheiten der Formel $-CH_2CH(C_{n-2}H_{2n-3})O-$, im Wesentlichen aus Alkylenoxid-Einheiten der Formel $-CH(C_{n-2}H_{2n-3})CH_2O-$, oder sowohl aus Alkylenoxid-Einheiten der Formel $-CH_2CH(C_{n-2}H_{2n-3})O-$ als auch aus Alkylenoxid-Einheiten der Formel "$CH(C_{n-2}H_{2n-3})CH_2O-$ zusammensetzen, wobei in letzterem Fall die beiden Alkylenoxid-Einheiten statistisch verteilt, alternierend oder in zwei oder mehreren Unterblöcken angeordnet sein können. Bei der basenkatalysierten Alkoxylierung erhält man überwiegend Alkylenoxid-Einheiten der Formel $-CH_2CH(C_{n-2}H_{2n-3})O-$, da der Angriff des Anions bevorzugt am sterisch weniger gehinderten sekundären Kohlenstoffatom des Alkylenoxids erfolgt. Üblich sind Molverhältnisse von mehr als 60:40, 70:30 oder 80:20, beispielsweise etwa 85:15, zugunsten von Alkylenoxid-Einheiten der Formel-$CH_2CH(C_{n-2}H_{2n-3})O-$.

Bei den alkoxylierten Alkoholen der Formel IIa handelt es sich um Alkoholalkoxylate mit einem Ethylenoxid-Block und einem höheren Alkylenoxid-Block, wobei der Ethylenoxid-Block terminal angeordnet ist, z.B. PO/EO-, BO/EO, PeO/EO oder DeO/PO-Blockalkoxylate.

Von den hier beschriebenen alkoxylierten Alkoholen sind insbesondere diejenigen bevorzugt, in denen n = 3 ist, die höhere Alkylenoxid-Einheit also eine Propylenoxid-Einheit ist. Hierzu gehören vor allem Alkoholblockalkoxylate der Formel (III)

$$R-O-(C_mH_{2m}O)_q-(C_3H_6O)_x-(C_2H_4O)_y-Z \qquad (III)$$

worin

R für $C_1$-$C_7$-Alkyl steht;
m für 2 oder 3 steht;
q für 0, 1, 2, oder 3 steht;
x für einen Wert von 1 bis 100 steht;
y für einen Wert von 1 bis 100 steht;
x+y einem Wert von 2 bis 100 entspricht; und
Z für Wasserstoff oder einen Endgruppenverschluß steht.

Dieser erfindungsgemäße Typ von Alkoholalkoxylat basiert auf mindestens einem Propylenoxidblock und mindestens einem Ethylenoxidblock, wobei der Ethylenoxidblock terminal angeordnet ist. Weitere besondere Ausgestaltungen ergeben sich aus den Ausführungen zu den Alkoholalkoxylaten der Formel (IIa).

Die erfindungsgemäßen Alkoholalkoxylate der Formel (III) weisen einem weiteren besonderen Aspekt der Erfindung zufolge mindestens oder mehr als 5, bevorzugt mindestens oder mehr als 8 insbesondere mindestens oder mehr als 12 Propylenoxid-Einheiten auf (Wert von x). Andererseits weisen die erfindungsgemäßen Alkoholalkoxylate der Formel (III) einem weiteren besonderen Aspekt der Erfindung zufolge höchstens oder weniger als 35, bevorzugt höchstens oder weniger als 25, und insbesondere höchstens oder weniger als 20 Propylenoxid-Einheiten auf (Wert von

x). Bevorzugt sind demnach beispielsweise Alkoholalkoxylate der Formel (IV) mit 5 bis 35, bevorzugt 8 bis 25, und insbesondere 12 bis 20 Propylenoxid-Einheiten (Wert von x).

Die erfindungsgemäßen Alkoholalkoxylate der Formel (III) weisen einem weiteren besonderen Aspekt der Erfindung zufolge mindestens oder mehr als 2, bevorzugt mindestens oder mehr als 3, insbesondere mindestens oder mehr als 5 Ethylenoxid-Einheiten auf (Wert von y). Andererseits weisen die erfindungsgemäßen Alkoholalkoxylate der Formel (III) einem weiteren besonderen Aspekt der Erfindung zufolge höchstens oder weniger als 30, bevorzugt höchstens oder weniger als 20, und insbesondere höchstens oder weniger als 12 Ethylenoxid-Einheiten auf (Wert von y). Bevorzugt sind demnach beispielsweise Alkoholalkoxylate der Formel (IV) mit 2 bis 30, bevorzugt 3 bis 20, und insbesondere 5 bis 12 Ethylenoxid-Einheiten (Wert von y).

Die erfindungsgemäßen Alkoholalkoxylate der Formel (III) weisen einem weiteren besonderen Aspekt der Erfindung zufolge einen Alkoxylatteil auf, im dem das Verhältnis von Ethylenoxid zu Propylenoxid (y zu x) wenigstens oder mehr als 1:5, bevorzugt wenigstens oder mehr als 1:4, und insbesondere wenigstens oder mehr als 1:3 beträgt. Andererseits weisen die erfindungsgemäßen Alkoholalkoxylate der Formel (III) einem weiteren besonderen Aspekt der Erfindung zufolge einen Alkoxylatteil auf, im dem das Verhältnis von Ethylenoxid zu Propylenoxid (y zu x) höchstens oder weniger als 2:1, bevorzugt höchstens oder weniger als 3:2, und insbesondere höchstens oder weniger als 1:1 beträgt. Bevorzugt sind demnach beispielsweise Alkoxylate der Formel (III), bei denen das Verhältnis von Ethylenoxid zu Propylenoxid (y zu x) 1:5 bis 2:1, vorzugsweise 1:4 bis 3:2 und insbesondere 1:3 bis 1:1 beträgt.

Von den hier beschriebenen alkoxylierten Alkoholen sind des Weiteren insbesondere diejenigen bevorzugt, in denen n = 4 ist, die höhere Alkylenoxid-Einheit also eine Butylenoxid-Einheit ist. Hierzu gehören vor allem Alkoholblockalkoxylate der Formel (IV)

$$R\text{-}O\text{-}(C_mH_{2m}O)_q\text{-}(C_4H_8O)_x\text{-}(C_2H_4O)_y\text{-}Z \qquad \text{(IV)}$$

worin

| | |
|---|---|
| R | für $C_1$-$C_7$-Alkyl steht; |
| m | für 2 oder 3 steht; |
| q | für 0, 1, 2, oder 3 steht; |
| x | für einen Wert von 1 bis 100 steht; |
| y | für einen Wert von 1 bis 100 steht; |
| x+y | einem Wert von 2 bis 100 entspricht; und |
| Z | für Wasserstoff oder einen Endgruppenverschluß steht. |

Dieser erfindungsgemäße Typ von Alkoholalkoxylat basiert auf einem Butylenoxidblock und mindestens einem Ethylenoxidblock, wobei der Ethylenoxidblock terminal angeordnet ist. Weitere besondere Ausgestaltungen ergeben sich aus den Ausführungen zu den Alkoholalkoxylaten der Formel (IIa).

Die erfindungsgemäßen Alkoholalkoxylate der Formel (IV) weisen einen hydrophoben Teil (R-O-$(C_mH_{2m}O)_q$-$(C_4H_8O)_x$-) und einen hydrophilen Teil (-$(C_2H_4O)_y$-Z) auf. Ist q größer als Null, so ist m vorzugsweise 3. Ein besonderer hydrophober Teil ergibt sich, wenn q Null ist (R-O-$(C_4H_8O)_x$-).

Gemäß einer besonderen Ausführungsform ist der hydrophobe Teil der erfindungsgemäßen Alkoholalkoxylate der Formel (IV) dadurch gekennzeichnet, dass er mindestens 15 Kohlenstoffatome im Rest R und der Gruppe -$(C_4H_8O)_x$-aufweist (sprich die Summe aus (4 • x) und der Anzahl der Kohlenstoffatome in R beträgt mindestens 15). Vorzugsweise weist der hydrophobe Teil der erfindungsgemäßen Alkoholalkoxylate wenigstens 17 und insbesondere wenigstens 19 Kohlenstoffatome auf. Andererseits ist der hydrophobe Teil der Alkoholalkoxylate einem weiteren Aspekt der Erfindung zufolge dadurch gekennzeichnet, dass er höchstens 60, vorzugsweise höchstens 55 oder 50, und insbesondere höchstens 46 Kohlenstoffatome aufweist.

Gemäß einer weiteren besonderen Ausführungsform ist der hydrophobe Teil der erfindungsgemäßen Alkoholalkoxylate der Formel (IV) dadurch gekennzeichnet, dass er mindestens 2 Verzweigungen aufweist. Wenigstens 1 Verzweigung liegt dabei im Alkoxylatteil. Andererseits ist der hydrophobe Teil der Alkoholalkoxylate einem weiteren Aspekt der Erfindung zufolge dadurch gekennzeichnet, dass er höchstens 12, vorzugsweise höchstens 11 oder 10, und insbesondere höchstens 9 Verzweigungen aufweist. Tatsächlich steuert jedes Butylenoxid eine Verzweigung bei, weshalb die Zahl an Verzweigungen im hydrophoben Alkoxylatteil der Summe der von den Butylenoxid-Einheiten beigesteuerten Kohlenstoffatome entspricht, d.h. (4 • x).

Gemäß einer weiteren besonderen Ausführungsform ist der hydrophobe Teil der erfindungsgemäßen Alkoholalkoxylate der Formel (IV) dadurch gekennzeichnet, dass er wenigstens 0,1, vorzugsweise wenigstens 0,13 und insbesondere wenigstens 0,16 Verzweigungen pro C-Atom aufweist. Andererseits ist der hydrophobe Teil der Alkoholalkoxylate einem weiteren Aspekt der Erfindung zufolge dadurch gekennzeichnet, dass er höchstens 0,3, vorzugsweise höchstens 0,27, und insbesondere höchstens 0,25 Verzweigungen aufweist.

Die erfindungsgemäßen Alkoholalkoxylate der Formel (IV) weisen einem weiteren besonderen Aspekt der Erfindung zufolge mindestens oder mehr als 2, bevorzugt mindestens oder mehr als 3, insbesondere mindestens oder mehr als 4,5 Butylenoxid-Einheiten auf (Wert von x). Andererseits weisen die erfindungsgemäßen Alkoholalkoxylate der Formel (IV) einem weiteren besonderen Aspekt der Erfindung zufolge höchstens oder weniger als 15, bevorzugt höchstens oder weniger als 12, und insbesondere höchstens oder weniger als 9 Butylenoxid-Einheiten auf (Wert von x). Bevorzugt sind demnach beispielsweise Alkoholalkoxylate der Formel (IV) mit 2 bis 15, bevorzugt 3 bis 12, und insbesondere 4,5 bis 9 Butylenoxid-Einheiten (Wert von x).

Die erfindungsgemäßen Alkoholalkoxylate der Formel (IV) weisen einem weiteren besonderen Aspekt der Erfindung zufolge mindestens oder mehr als 2, bevorzugt mindestens oder mehr als 3, und insbesondere mindestens oder mehr als 5 Ethylenoxid-Einheiten auf (Wert von y). Andererseits weisen die erfindungsgemäßen Alkoholalkoxylate der Formel (IV) einem weiteren besonderen Aspekt der Erfindung zufolge höchstens oder weniger als 30, bevorzugt höchstens oder weniger als 20, und insbesondere höchstens oder weniger als 12 Ethylenoxid-Einheiten auf (Wert von y). Bevorzugt sind demnach beispielsweise Alkoholalkoxylate der Formel (IV) mit 2 bis 30, bevorzugt 3 bis 20, und insbesondere 5 bis 12 Ethylenoxid-Einheiten (Wert von y).

Die erfindungsgemäßen Alkoholalkoxylate der Formel (IV) weisen einem weiteren besonderen Aspekt der Erfindung zufolge einen Alkoxylatteil auf, im dem das Verhältnis von Ethylenoxid zu Butylenoxid (y zu x) wenigstens oder mehr als 1:4, bevorzugt wenigstens oder mehr als 1:3, und insbesondere wenigstens oder mehr als 1:2 beträgt. Andererseits weisen die erfindungsgemäßen Alkoholalkoxylate der Formel (IV) einem weiteren besonderen Aspekt der Erfindung zufolge einen Alkoxylatteil auf, im dem das Verhältnis von Ethylenoxid zu Butylenoxid (y zu x) höchstens oder weniger als 8:1, bevorzugt höchstens oder weniger als 5:1, und insbesondere höchstens oder weniger als 3:1 beträgt. Bevorzugt sind demnach beispielsweise Alkoxylate der Formel (IV), bei denen das Verhältnis von Ethylenoxid zu Butylenoxid (y zu x) 1:4 bis 8:1, vorzugsweise 1:3 bis 5:1, und insbesondere 1:2 bis 3:1 beträgt.

Von den hier beschriebenen alkoxylierten Alkoholen sind des Weiteren insbesondere diejenigen bevorzugt, in denen n = 5 ist, die höhere Alkylenoxid-Einheit also eine Pentylenoxid-Einheit ist. Hierzu gehören vor allem Alkoholblockalkoxylate der Formel (V)

$$R\text{-}O\text{-}(C_mH_{2m}O)_q\text{-}(C_5H_{10}O)_x\text{-}(C_2H_4O)_y\text{-}Z \qquad (V)$$

worin

- R für $C_1$-$C_7$-Alkyl steht;
- m für 2 oder 3 steht;
- q für 0, 1, 2, oder 3 steht;
- x für einen Wert von 1 bis 100 steht;
- y für einen Wert von 1 bis 100 steht;
- x+y einem Wert von 2 bis 100 entspricht; und
- Z für Wasserstoff oder einen Endgruppenverschluß steht.

Dieser erfindungsgemäße Typ von Alkoholalkoxylat basiert auf einem Pentylenoxidblock und mindestens einem Ethylenoxidblock, wobei der Ethylenoxidblock terminal angeordnet ist. Weitere besondere Ausgestaltungen ergeben sich aus den Ausführungen zu den Alkoholalkoxylaten der Formel (IIa).

Auch die erfindungsgemäßen Alkoholalkoxylate der Formel (V) weisen einen hydrophoben Teil (R-O-$(C_mH_{2m}O)_q$-$(C_5H_{10}O)_x$-) und einen hydrophilen Teil (-$(C_2H_4O)_y$-Z) auf. Ist q größer als Null, so ist m vorzugsweise 3. Ein besonderer hydrophober Teil ergibt sich, wenn q Null ist (R-O-$(C_5H_{10}O)_x$-).

Gemäß einer besonderen Ausführungsform ist der hydrophobe Teil der erfindungsgemäßen Alkoholalkoxylate der Formel (V) dadurch gekennzeichnet, dass er mindestens 15 Kohlenstoffatome im Rest R und der Gruppe -$(C_5H_{10}O)_x$-aufweist (sprich die Summe aus (5 • x) und der Anzahl der Kohlenstoffatome in R beträgt mindestens 15). Vorzugsweise weist der hydrophobe Teil der erfindungsgemäßen Alkoholalkoxylate wenigstens 17 und insbesondere wenigstens 19 Kohlenstoffatome auf. Andererseits ist der hydrophobe Teil der Alkoholalkoxylate einem weiteren Aspekt der Erfindung zufolge dadurch gekennzeichnet, dass er höchstens 60, vorzugsweise höchstens 55 oder 50, und insbesondere höchstens 46 Kohlenstoffatome aufweist.

Gemäß einer weiteren besonderen Ausführungsform ist der hydrophobe Teil der erfindungsgemäßen Alkoholalkoxylate der Formel (V) dadurch gekennzeichnet, dass er mindestens 2 Verzweigungen aufweist. Wenigstens 1 Verzweigung liegt dabei im Alkoxylatteil. Andererseits ist der hydrophobe Teil der Alkoholalkoxylate einem weiteren Aspekt der Erfindung zufolge dadurch gekennzeichnet, dass er höchstens 12, vorzugsweise höchstens 11 oder 10, und insbesondere höchstens 9 Verzweigungen aufweist. Tatsächlich steuert jedes Pentylenoxid eine Verzweigung bei, weshalb die Zahl an Verzweigungen im hydrophoben Alkoxylatteil der Summe der von den Pentylenoxid-Einheiten beigesteuerten Kohlenstoffatome entspricht, d.h. (5 • x).

Gemäß einer weiteren besonderen Ausführungsform ist der hydrophobe Teil der erfindungsgemäßen Alkoholalkoxylate der Formel (V) dadurch gekennzeichnet, dass er wenigstens 0,1, vorzugsweise wenigstens 0,13 und insbesondere wenigstens 0,16 Verzweigungen pro C-Atom aufweist. Andererseits ist der hydrophobe Teil der Alkoholalkoxylate einem weiteren Aspekt der Erfindung zufolge dadurch gekennzeichnet, dass er höchstens 0,3, vorzugsweise höchstens 0,27, und insbesondere höchstens 0,25 Verzweigungen aufweist.

Die erfindungsgemäßen Alkoholalkoxylate der Formel (V) weisen einem weiteren besonderen Aspekt der Erfindung zufolge mindestens oder mehr als 2, bevorzugt mindestens oder mehr als 3, insbesondere mindestens oder mehr als 4 Pentylenoxid-Einheiten auf (Wert von x). Andererseits weisen die erfindungsgemäßen Alkoholalkoxylate der Formel (V) einem weiteren besonderen Aspekt der Erfindung zufolge höchstens oder weniger als 15, bevorzugt höchstens oder weniger als 12, und insbesondere höchstens oder weniger als 9 Pentylenoxid-Einheiten auf (Wert von x). Bevorzugt sind demnach beispielsweise Alkoholalkoxylate der Formel (V) mit 2 bis 15, bevorzugt 3 bis 12, und insbesondere 4 bis 9 Pentylenoxid-Einheiten (Wert von x).

Die erfindungsgemäßen Alkoholalkoxylate der Formel (V) weisen einem weiteren besonderen Aspekt der Erfindung zufolge mindestens oder mehr als 2, bevorzugt mindestens oder mehr als 3, insbesondere mindestens oder mehr als 5 Ethylenoxid-Einheiten auf (Wert von y). Andererseits weisen die erfindungsgemäßen Alkoholalkoxylate der Formel (V) einem weiteren besonderen Aspekt der Erfindung zufolge höchstens oder weniger als 30, bevorzugt höchstens oder weniger als 20, und insbesondere höchstens oder weniger als 12 Ethylenoxid-Einheiten auf (Wert von y). Bevorzugt sind demnach beispielsweise Alkoholalkoxylate der Formel (V) mit 2 bis 30, bevorzugt 3 bis 20, und insbesondere 5 bis 12 Ethylenoxid-Einheiten (Wert von y).

Die erfindungsgemäßen Alkoholalkoxylate der Formel (V) weisen einem weiteren besonderen Aspekt der Erfindung zufolge einen Alkoxylatteil auf, im dem das Verhältnis von Ethylenoxid zu Pentylenoxid (y zu x) wenigstens oder mehr als 1:4, bevorzugt wenigstens oder mehr als 1:3, und insbesondere wenigstens oder mehr als 1:2 beträgt. Andererseits weisen die erfindungsgemäßen Alkoholalkoxylate der Formel (V) einem weiteren besonderen Aspekt der Erfindung zufolge einen Alkoxylatteil auf, im dem das Verhältnis von Ethylenoxid zu Pentylenoxid (y zu x) höchstens oder weniger als 8:1, bevorzugt höchstens oder weniger als 5:1, und insbesondere höchstens oder weniger als 3:1 beträgt. Bevorzugt sind demnach beispielsweise Alkoxylate der Formel (V), bei denen das Verhältnis von Ethylenoxid zu Pentylenoxid (y zu x) 1:4 bis 8:1, vorzugsweise 1:3 bis 3:1 und insbesondere 1:2 bis 5:1 beträgt.

Von den hier beschriebenen alkoxylierten Alkoholen sind des Weiteren insbesondere diejenigen bevorzugt, in denen n = 10 ist, die höhere Alkylenoxid-Einheit also eine Decylenoxid-Einheit ist. Hierzu gehören vor allem Alkoholblockalkoxylate der Formel (VI)

$$R\text{-}O\text{-}(C_mH_{2m}O)_q\text{-}(C_{10}H_{20}O)_x\text{-}(C_2H_4O)_y\text{-}Z \qquad (VI)$$

worin

- R für $C_1$-$C_7$-Alkyl steht;
- m für 2 oder 3 steht;
- q für 0, 1, 2, oder 3 steht;
- x für einen Wert von 1 bis 100 steht;
- y für einen Wert von 1 bis 100 steht;
- x+y einem Wert von 2 bis 100 entspricht; und
- Z für Wasserstoff oder einen Endgruppenverschluß steht.

Dieser erfindungsgemäße Typ von Alkoholalkoxylat basiert auf einem Decylenoxidblock und mindestens einem Ethylenoxidblock, wobei der Ethylenoxidblock terminal angeordnet ist. Weitere besondere Ausgestaltungen ergeben sich aus den Ausführungen zu den Alkoholalkoxylaten der Formel (IIa).

Auch die erfindungsgemäßen Alkoholalkoxylate der Formel (VI) weisen einen hydrophoben Teil ($R\text{-}O\text{-}(C_mH_{2m}O)_q\text{-}(C_{10}H_{20}O)_x\text{-}$) und einen hydrophilen Teil ($\text{-}(C_2H_4O)_y\text{-}Z$) auf. Ist q größer als Null, so ist m vorzugsweise 3. Ein besonderer hydrophober Teil ergibt sich, wenn q Null ist ($R\text{-}O\text{-}(C_{10}H_{20}O)_x\text{-}$).

Gemäß einer besonderen Ausführungsform ist der hydrophobe Teil der erfindungsgemäßen Alkoholalkoxylate der Formel (VI) dadurch gekennzeichnet, dass er mindestens 15 Kohlenstoffatome im Rest R und der Gruppe $\text{-}(C_{10}H_{20}O)_x$-aufweist (sprich die Summe aus (10 • x) und der Anzahl der Kohlenstoffatome in R beträgt mindestens 15). Vorzugsweise weist der hydrophobe Teil der erfindungsgemäßen Alkoholalkoxylate wenigstens 17 und insbesondere wenigstens 19 Kohlenstoffatome auf. Andererseits ist der hydrophobe Teil der Alkoholalkoxylate einem weiteren Aspekt der Erfindung zufolge dadurch gekennzeichnet, dass er höchstens 60, vorzugsweise höchstens 55 oder 50, und insbesondere höchstens 46 Kohlenstoffatome aufweist.

Gemäß einer weiteren besonderen Ausführungsform ist der hydrophobe Teil der erfindungsgemäßen Alkoholalkoxylate der Formel (VI) dadurch gekennzeichnet, dass er mindestens 2 Verzweigungen aufweist. Wenigstens 1

Verzweigung liegt dabei im Alkoxylatteil. Andererseits ist der hydrophobe Teil der Alkoholalkoxylate einem weiteren Aspekt der Erfindung zufolge dadurch gekennzeichnet, dass er höchstens 12, vorzugsweise höchstens 11 oder 10, und insbesondere höchstens 9 Verzweigungen aufweist. Tatsächlich steuert jedes Decylenoxid eine Verzweigung bei, weshalb die Zahl an Verzweigungen im hydrophoben Alkoxylatteil der Summe der von den Decylenoxid-Einheiten beigesteuerten Kohlenstoffatome entspricht, d.h. (10 • x).

Gemäß einer weiteren besonderen Ausführungsform ist der hydrophobe Teil der erfindungsgemäßen Alkoholalkoxylate der Formel (VI) dadurch gekennzeichnet, dass er wenigstens 0,1, vorzugsweise wenigstens 0,13 und insbesondere wenigstens 0,16 Verzweigungen pro C-Atom aufweist. Andererseits ist der hydrophobe Teil der Alkoholalkoxylate einem weiteren Aspekt der Erfindung zufolge dadurch gekennzeichnet, dass er höchstens 0,3, vorzugsweise höchstens 0,27, und insbesondere höchstens 0,25 Verzweigungen aufweist.

Die erfindungsgemäßen Alkoholalkoxylate der Formel (VI) weisen einem weiteren besonderen Aspekt der Erfindung zufolge mindestens oder mehr als 1, bevorzugt mindestens oder mehr als 1,5 insbesondere mindestens oder mehr als 2 Decylenoxid-Einheiten auf (Wert von x). Andererseits weisen die erfindungsgemäßen Alkoholalkoxylate der Formel (VI) einem weiteren besonderen Aspekt der Erfindung zufolge höchstens oder weniger als 5, bevorzugt höchstens oder weniger als 4, und insbesondere höchstens oder weniger als 3 Decylenoxid-Einheiten auf (Wert von x). Bevorzugt sind demnach beispielsweise Alkoholalkoxylate der Formel (VI) mit 1 bis 5, bevorzugt 1,5 bis 4, und insbesondere 2 bis 3 Decylenoxid-Einheiten (Wert von x).

Die erfindungsgemäßen Alkoholalkoxylate der Formel (VI) weisen einem weiteren besonderen Aspekt der Erfindung zufolge mindestens oder mehr als 2, bevorzugt mindestens oder mehr als 3, insbesondere mindestens oder mehr als 5 Ethylenoxid-Einheiten auf (Wert von y). Andererseits weisen die erfindungsgemäßen Alkoholalkoxylate der Formel (VI) einem weiteren besonderen Aspekt der Erfindung zufolge höchstens oder weniger als 30, bevorzugt höchstens oder weniger als 20, und insbesondere höchstens oder weniger als 12 Ethylenoxid-Einheiten auf (Wert von y). Bevorzugt sind demnach beispielsweise Alkoholalkoxylate der Formel (V) mit 2 bis 30, bevorzugt 3 bis 20, und insbesondere 5 bis 12 Ethylenoxid-Einheiten (Wert von y).

Die erfindungsgemäßen Alkoholalkoxylate der Formel (VI) weisen einem weiteren besonderen Aspekt der Erfindung zufolge einen Alkoxylatteil auf, im dem das Verhältnis von Ethylenoxid zu Decylenoxid (y zu x) wenigstens oder mehr als 1:1, bevorzugt wenigstens oder mehr als 3:1, und insbesondere wenigstens oder mehr als 5:1 beträgt. Andererseits weisen die erfindungsgemäßen Alkoholalkoxylate der Formel (VI) einem weiteren besonderen Aspekt der Erfindung zufolge einen Alkoxylatteil auf, im dem das Verhältnis von Ethylenoxid zu Decylenoxid (y zu x) höchstens oder weniger als 30:1 bevorzugt höchstens oder weniger als 15:1, und insbesondere höchstens oder weniger als 10:1 beträgt. Bevorzugt sind demnach beispielsweise Alkoxylate der Formel (VI), bei denen das Verhältnis von Ethylenoxid zu Decylenoxid (y zu x) 1:1 bis 30:1, vorzugsweise 3:1 bis 15:1 und insbesondere 5:1 bis 10:1 beträgt.

Eine besondere Ausführungsform sind Mittel, umfassend Alkoholalkoxylate, die nicht endgruppenverschlossen sind (Z = H).

Einer weiteren besonderen Asuführungsform zufolge sind die erfindungsgemäßen Alkoholalkoxylate endgruppenverschlossen. In diesem Fall steht Z vorzugsweise für $C_1$-$C_4$-Alkyl, noch bevorzugter für $C_1$-$C_3$-Alkyl und insbesondere für Methyl. Ferner kommen für Z auch Reste wie $C_2$-$C_4$-Alkenyl (beispielsweise Allyl), $C_6$-$C_{10}$-Aryl (beispielsweise Phenyl) oder $C_6$-$C_{10}$-Aryl-$C_1$-$C_2$-alkyl (beispielsweise Benzyl), $C_1$-$C_4$-Alkylcarbonyl (beispielsweise Acetyl, Propionyl, Butyryl), $C_1$-$C_{10}$-Arylcarbonyl (beispielsweise Benzoyl) in Frage. Auch tertiäre Alkoholreste wie 2-Hydroxyisobutyl oder anorganische Säuregruppen, insbesondere Phosphat, Diphosphat oder Sulfat, sind geeignet.

Engruppenverschiossene Alkoholalkoxylate können in an sich bekannter Weise hergestellt werden, indem man das nicht engruppenverschlossene Alkoholalkoxylat mit geeigneten Reagenzien, beispielsweise Dialkylsulfaten, umsetzt. Derartige Umsetzungen sind beispielsweise in EP-A 0 302 487 und EP-A 0 161 537 beschrieben. Auf die dortigen Ausführungen wird vollumfänglich Bezug genommen.

Das theoretische Molekulargewicht erfindungsgemäß geeigneter Alkoholalkoxylate beträgt in der Regel weniger als 2000 g/mol. Bevorzugt sind Alkoholalkoxylate mit einem Molekulargewicht von weniger als 1800 g/mol, weniger als 1700 g/mol, oder weniger als 1500 g/mol. Einer besonderen Ausführungsform zufolge beträgt das Molekulargewicht weniger als 1400 g/mol.

Das gewichtsmittlere Molekulargewicht erfindungsgemäß geeigneter Alkoholalkoxylate beträgt in der Regel weniger als 2000 g/mol. Bevorzugt sind Alkoholalkoxylate mit einem Molekulargewicht von weniger als 1800 g/mol, weniger als 1700 g/mol, oder weniger als 1500 g/mol. Einer besonderen Ausführungsform zufolge beträgt das Molekulargewicht weniger als 1400 g/mol. Die Angaben zu den gewichtsmittleren Molekulargewicht beziehen auf die Bestimmung mittels Gelpermeationschromatographie nach DIN 55672.

Der Begriff "Verzweigungsgrad" von R wird hier in prinzipiell bekannter Art und Weise für die Zahl der Methylgruppen in R abzüglich 1 definiert. Für Z gilt Analoges. Der Verzweigungsgrad des Alkoxylatteils ergibt sich aus dem Alkoxylierungsgrad und den an der Alkoxylierung beteiligten Alkylenoxiden. Der mittlere Verzweigungsgrad ist der statistische Mittelwert der Verzweigungsgrade aller Moleküle einer Probe.

Der mittlere Verzweigungsgrad kann für primäre und/oder sekundäre Alkohole wie folgt $^1$H-NMR-spektrosko-

pisch ermittelt werden: Dazu wird eine Probe des Alkohols zunächst einer Derivatisierung mit Trichloracetylisocyanat (TAI) unterzogen. Dabei werden die Alohole in die Carbaminsäureester überführt. Die Signale der veresterten primären Alkohole liegen bei $\delta$= 4,7 bis 4,0 ppm, die der veresterten sekundären Alkohole bei etwa 5 ppm und in der Probe vorhandenes Wasser reagiert mit TAI zur Carbaminsäure ab. Alle Methyl-, Methylen- und Methinprotonen liegen im Bereich von 2,4 bis 0,4 ppm. Die Signale < 1 ppm sind dabei den Methylgruppen zugeordnet. Aus dem so erhalten Spektrum läßt sich der mittlere Verzweigungsgrad (Iso-Index) wie folgt berechnen:

$$\text{Iso-Index} = ((F(CH_3) / 3) / (F(CH_2\text{-}OH) / 2 + F(CHR\text{-}OH))) - 1$$

wobei $F(CH_3)$ für die den Methylprotonen entsprechende Signalfläche, $F(CH_2\text{-}OH)$ für die Signalfläche der Methylenprotonen in der $CH_2$-OH-Gruppe und F(CHR-OH) für die Signalfläche der Methinprotonen in der CHR-OH-Gruppe steht.

Anteile der Komponente (b), d.h. an Alkoholalkoxylat, am Gesamtgewicht des erfindungsgemäßen Mittels von mehr als 1 Gew.-%, vorzugsweise von mehr als 5 Gew.-% und insbesondere von mehr als 10 Gew.-% sind von Vorteil. Andererseits sind Anteile der Komponente (b) am Gesamtgewicht des Mittels von weniger als 50 Gew.-%, vorzugsweise von weniger als 45 Gew.-% und insbesondere von weniger als 40 Gew.-% in der Regel zweckmäßig.

Als Pflanzenbehandlungswirkstoff der Komponente (a) kann jede Substanz bezeichnet werden, deren Zweck bzw. Wirkung es ist, dem Befall einer Pflanze durch irgendeinen Schädling vorzubeugen oder den Schädling abzuwehren, abzuschrecken, zu vernichten oder auf andere Weise den von ihm verursachten Schaden zu verringern (Pestizid). Wie eingangs gesagt, können Pflanzenschädlinge zu verschiedenen Gruppen von Lebewesen gehören; unter den höheren Tieren sind insbesondere unter Insekten und Milben zahlreiche wichtige Schädlinge zu finden, ferner unter Nematoden und Schnecken; Wirbeltiere wie Säuger und Vögel sind in den Industrieländern heute von untergeordneter Bedeutung. Zahlreiche Gruppen von Mikroben, darunter Pilze, Bakterien einschließlich der Mykoplasmen, Viren und Viroide umfassen Schädlinge, und auch Unkräuter, die mit Nutzpflanzen um knappen Lebensraum und andere Ressourcen konkurrieren, können zu den Schädlingen im weiteren Sinne gerechnet werden. Pestizide umfasen insbesondere Avizide, Acarizide, Austrocknungsmittel, Bakterizide, Chemosterilisatoren, Entlaubungsmittel, Fraßhemmer, Fungizide, Herbizide, Herbizidsicherungsstoffe, Insektenlockstoffe, Insektizide, Insektenabschreckmittel, Molluskizide, Nematizide, Paarungsverhinderer (mating disrupters), Pflanzenaktivatoren, Pflanzenwachstumsregulatoren, Rodentizide, Säugerabschreckmittel, Synergisten, Vogelabschreckmittel und Viruzide.

Aufgeschlüsselt nach chemischen Klassen umfassen Pestizide insbesondere Acylalaninfungizide, Acylaminosäurefungizide, aliphatische Amid-Organothiophosphatinsektizide, aliphatische Organothiophosphatinsektizide, aliphatische Stickstofffungizide, Amidfungizide, Amidherbizide, Anilidfungizide, Anilidherbizide, anorganische Fungizide, anorganische Herbizide, anorganische Rodentizide, Antiauxine, Antibiotika-Acarizide, Antibiotikafungizide, Antibiotikaherbizide, Antibiotikainsektizide, Antibiotikanematizide, Aromatensäurenfungizide, Aromatensäurenherbizide, Arsenherbizide, Arseninsektizide, Arylalaninherbizide, Aryloxyphenoxypropionsäureherbizide, Auxine, Avermectinacarizide, Avermectininsektizide, Benzamidfungizide, Benzanilidfungizide, Benzimidazolfungizide, Benzimidazolvorläuferfungizide, Benzimidazolylcarbamatfungizide, Benzoesäureherbizide, Benzofuranylalkylsulfonatherbizide, Benzofuranylmethylcarbamatinsektizide, Benzothiazolfungizide, Benzothiopyranorganothiophosphatinsektizide, Benzotriazineorganothiophosphatinsektizide, Benzoylcyclohexandionherbizide, Bipyridyliumherbizide, Brückendiphenylacarizide, Brückendiphenylfungizide, Carbamatacarizide, Carbamatfungizide, Carbamatherbizide, Carbamatinsektizide, Carbamatnematizide, Carbanilatfungizide, Carbanilatherbizide, Chinolinecarboxylatherbizide, Chinolinfungizide, Chinonfungizide, Chinoxalinacarizide, Chinoxalineorganothiophosphatinsektizide, Chinoxalinfungizide, Chitinsyntheseinhibitoren, Chloracetanilidherbizide, Chlornicotinylinsektizide, Chlorpyridinherbizide, Chlortriazinherbizide, Conazolfungizide, Cumarinrodentizide, Cyclodithiocarbamatfungizide, Cyclohexenoximherbizide, Cyclopropylisoxazolherbizide, Cytokinine, Diacylhydrazininsektizide, Dicarboximidfungizide, Dicarboximidherbizide, Dichlorphenyldicarboximidfungizide, Dimethylcarbamatinsektizide, Dinitroanilinherbizide, Dinitrophenolacarizide, Dinitrophenolfungizide, Dinitrophenolherbizide, Dinitrophenolinsektizide, Diphenyletherherbizide, Dithiocarbamatfungizide, Dithiocarbamatherbizide, Entlaubungsmittel, Ethylenfreisetzer, Fluorinsektizide, Furamidfungizide, Furanilidfungizide, Gibberelline, halogenierte aliphatische Herbizide, Harnstofffungizide, Harnstoffherbizide, Harnstoffinsektizide, Harnstoffrodentizide, Häutungshormone, Häutungshormonmimetika, Häutungsverhinderer, heterocyclische Organothiophosphatinsektizide, Imidazolfungizide, Imidazolinonherbizide, Indandionrodentizide, Insektenwachstumsregulatoren, Isoindolorganothiophosphatinsektizide, Isoxazolorganothiophosphatinsektizide, Juvenilhormone, Juvenilhormonmimetika, Kupferfungizide, macrocyclische Lactonacarizide, macrocyclische Lactoninsektizide, Methoxytriazinherbizide, Methylthiotriazinherbizide, Milbemycinacarizide, Milbemycininsektizide, Milbenwachstumsregulatoren, Morphactine, Morpholinfungizide, Nereistoxinanaloga, Nicotinoidinsektizide, Nitrilherbizide, Nitroguanidininsektizide, Nitromethyleninsektizide, Nitrophenyletherherbizide, Organochlorinacarizide, Organochlorininsektizide, Organochlorinrodentizide, Organophosphatacarizide, Organophosphatinsektizide, Organophosphatnematizide, Organophosphoracarizide, Organophosphorfungizide, Organophosphorherbizide, Organophosphorinsektizide, Organophosphornematizide, Organophosphor-Rodentizide, Organothiophosphatacarizide, Organothiophosphatinsektizide, Organothiophosphatnematizide, Organotinacarizide, Organozinnfungizide, Oxadiazininsektizide, Oxathiinfungizide, Oxazolfungizide, Oximcarbamatacarizide, Oximcarbamatnematizide, Oximcarbamatinsektizide, Oximorganothiophosphatinsektizide, pflanzliche Insektizide, pflanzliche Rodentizide, Phenoxybuttersäureherbizide, Phenoxyessigsäureherbizide, Phenoxyherbizide, Phenoxypropionsäureherbizide, Phenylendiaminherbizide, Phenylethylphosphonothioatinsektizide, Phenylharnstoffherbizide, Phenylmethylcarbamatinsektizide, Phenylorganothiophosphatinsektizide, Phenylphenylphosphonothioatinsektizide, Phenylpyrazolylketonherbizide, Phenylsulfamidacarizide, Phenylsulfamidfungizide, Phosphonatacarizide, Phosphonatinsektizide, Phosphonothioatinsektizide, Phosphoramidatinsektizide, Phosphoramidothioatacarizide, Phosphoramidothioatinsektizide, Phosphorodiamidacarizide, Phosphorodiamidinsektizide, Phthalatherbizide, Phthalimidacarizide, Phthalimidfungizide, Phthalimidinsektizide, Picolatherbizide, polymere Dithiocarbamatfungizide, Polysulfidfungizide, Precocene, Pyrazolacarizide, Pyrazolfungizide, Pyrazolinsektizide, Pyrazolopyrimidineorganothiophosphatinsektizide, Pyrazolyloxyacetophenonherbizide, Pyrazolylphenylherbizide, Pyrethroidacarizide, Pyrethroidesteracarizide, Pyrethroidesterinsektizide, Pyrethroidetheracarizide, Pyrethroidetherinsektizide, Pyrethroidinsektizide, Pyridazinherbizide, Pyridazinonherbizide, Pyridinfungizide, Pyridinherbizide, Pyridinorganothiophosphatinsektizide, Pyridylmethylamininsektizide, Pyrimidinaminacarizide, Pyrimidinamininsektizide, Pyrimidinaminrodentizide, Pyrimidinediaminherbizide, Pyrimidineorganothiophosphatinsektizide, Pyrimidinfungizide, Pyrimidinyloxybenzoesäureherbizide, Pyrimidinylsulfonylharnstoffherbizide, Pyrimidinylthiobenzoesäureherbizide, Pyrrolacarizide, Pyrrolfungizide, Pyrrolinsektizide, quaternäre Ammoniumherbizide, Strobilurinfungizide, Sulfitesteracarizide, Sulfonamidfungizide, Sulfonamidherbizide, Sulfonanilidfungizide, Sulfonanilidherbizide, Sulfonylharnstoffherbizide, Tetrazinacarizide, Tetronatacarizide, Tetronatinsektizide, Thiadiazoleorganothiophosphatinsektizide, Thiadiazolylharnstoffherbizide, Thiazolfungizide, Thiocarbamatacarizide, Thiocarbamatfungizide, Thiocarbamatherbizide, Thiocarbonatherbizide, Thioharnstoffacarizide, Thioharnstoffherbizide, Thioharnstoff-Rodentizide, Thiophenfungizide, Triazinfungizide, Triazinherbizide, Triazinonherbizide, Triazinylsulfonylharnstoffherbizide, Triazolfungizide, Triazolherbizide, Triazolonherbizide, Triazolopyrimidinfungizide, Triazolopyrimidinherbizide, Triazolorganothiophosphatinsektizide, Uracilherbizide, Valinamidfungizide, Wachstumsinhibitoren, Wachstumsstimulatoren, Wachstumsverzögerer, Xylylalaninfungizide.

Das Pestizid zur erfindungsgemäßen Verwendung ist insbesondere unter Fungiziden (a1), Herbiziden (a2) and Insektiziden (a3) ausgewählt.

Fungizide umfassen beispielsweise aliphatische Stickstofffungizide, wie Butylamin, Cymoxanil, Dodicin, Dodine, Guazatine, Iminoctadine; Amidfungizide, wie Carpropamid, Chloraniformethan, Cyflufenamid, Diclocymet, Ethaboxam, Fenoxanil, Flumetover, Furametpyr, Mandipropamid, Penthiopyrad, Prochloraz, Chinazamid, Silthiofam, Triforine; insbesondere Acylaminosäurefungizide, wie Benalaxyl, Benalaxyl-M, Furalaxyl, Metalaxyl, Metalaxyl-M, Pefurazoate; Anilidfungizide, wie Benalaxyl, Benalaxyl-M, Boscalid, Carboxin, Fenhexamid, Metalaxyl, Metalaxyl-M, Metsulfovax, Ofurace, Oxadixyl, Oxycarboxin, Pyracarbolid, Thifluzamide, Tiadinil; insbesondere Benzanilidfungizide, wie Benodanil, Flutolanil, Mebenil, Mepronil, Salicylanilide, Tecloftalam; Furanilidfungizide, wie Fenfuram, Furalaxyl, Furcarbanil, Methfuroxam; und Sulfonanilidfungizide, wie Flusulfamide; Benzamidfungizide, wie Benzohydroxamsäure, Fluopicolide, Tioxymid, Trichlamide, Zarilamid, Zoxamide; Furamidfungizide, wie Cyclafuramid, Furmecyclox; Phenylsulfamidfungizide, wie Dichlofluanid, Tolylfluanid; Sulfonamidfungizide, wie Cyazofamid; und Valinamidfungizide, wie Benthiavalicarb, Iprovalicarb; Antibiotika-Fungizide, wie Aureofungin, Blasticidin-S, Cycloheximide, Griseofulvin, Kasugamycin, Natamycin, Polyoxins, Polyoxorim, Streptomycin, Validamycin; insbesondere Strobilurinfungizide, wie Azoxystrobin, Dimoxystrobin, Fluoxastrobin, Kresoxim-Methyl, Metominostrobin, Orysastrobin, Picoxystrobin, Pyraclostrobin, Trifloxystrobin; aromatische Fungizide, wie Biphenyl, Chlordinitronaphthalen, Chloroneb, Chlorothalonil, Cresol, Dicloran, Chintozene, Tecnazene; Benzimidazolfungizide, wie Benomyl, Carbendazim, Chlorfenazol, Cypendazol, Debacarb, Fuberidazol, Mecarbinzid, Rabenzazol, Thiabendazole; Benzimidazolprecursorfungizide, wie Furophanate, Thiophanate, Thiophanat-Methyl; Benzothiazolfungizide, wie Bentaluron, Chlobenthiazon, TCMTB; Brückendiphenylfungizide, wie Bithionol, Dichlorphen, Diphenylamine; Carbamatfungizide, wie Benthiavalicarb, Furophanat, Iprovalicarb, Propamocarb, Thiophanat, Thiophanat-Methyl; insbesondere Benzimidazolylcarbamatfungizide, wie Benomyl, Carbendazim, Cypendazol, Debacarb, Mecarbinzid; und Carbanilatfungizide, wie Diethofencarb; Conazolfungizide; insbesondere Imidazole, wie Climbazol, Clotrimazol, Imazalil, Oxpoconazol, Prochloraz, Triflumizole; und Triazole, wie Azaconazol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-Cis, Hexaconazol, Imibenconazol, Ipconazol, Metconazol, Myclobutanil, Penconazol, Propiconazol, Prothioconazol, Chinconazol, Simeconazol, Tebuconazol, Tetraconazol, Triadimefon, Triadimenol, Triticonazol, Uniconazol, Uniconazol-P; Kupferfungizide, wie Bordeauxmischung, Burgundermischung, Cheshuntmischung, Kupferazetat, Kupferkarbonat, Kupferhydroxyd, Kupfernaphtenat, Kupferroleat, Kupferoxychlorid, Kupfersulfat, Kupferzinkchromat, Kupferoxyd, Mancopper, Cufraneb, Cuprobam, Oxinecopper; Dicarboximidfungizide, wie Famoxadon, Fluoroimide; insbesondere Dichlorphenyldicarboximidfungizide, wie Chlozolinate, Dichlozoline, Iprodion, Isovaledion, Myclozolin, Procymidon, Vinclozolin; und Phthalimidfungizide, wie Captafol, Captan, Ditalimfos, Folpet, Thiochlorfenphim; Dinitrophenolfungizide, wie Binapacryl, Dinobuton, Dinocap, Dinocap-4, Dinocap-6, Dinocton, Dinopenton, Dinosulfon, Dinoterbon, DNOC; Dithiocarbamatfungizide, wie Azithiram, Carbamorph, Cufraneb, Cuprobam, Disulfiram, Ferbam, Metam, Nabam, Tecoram, Thiram, Ziram; insbesondere Cyclodithiocarbamatfungizide, wie Dazomet, Etem, Milneb; und polymere Dithiocarbamatfungizide, wie Mancopper, Mancozeb, Maneb, Metiram, Polycarbamate, Propineb, Zineb; Imidazolfungizide, wie Cyazofamid, Fenamidon, Fenapanil, Glyodin, Iprodion, Isovaledion, Pefurazoate, Triazoxide; anorganische Fungizide, wie Kaliumazid, Natriumazid, Schwefel; Morpholinfungizide, wie z.B. Aldimorph, Benzamorph, Carbamorph, Dimethomorph, Dodemorph, Fenpropimorph, Flumorph, Tridemorph; Organophosphorfungizide, wie Ampropylfos, Ditalimfos, Edifenphos, Fosetyl, Hexylthiofos, Iprobenfos, Phosdiphen, Pyrazophos, Tolclofos-Methyl, Triamiphos; Organotinfungizide, wie Decafentin, Fentin, Tributyltinoxide; Oxathiinfungizide, wie Carboxin, Oxycarboxin; Oxazolfungizide, wie Chlozolinate, Dichlozoline, Drazoxolon, Famoxadon, Hymexazol, Metazoxolon, Myclozolin, Oxadixyl, Vinclozolin; Polysulfidfungizide, wie Bariumpolysulfid, Kaliumpolysulfid, Natriumpolysulfid; Pyrazolfungizide, wie Furametpyr, Penthiopyrad; Pyridinfungizide, wie Boscalid, Buthiobate, Dipyrithion, Fluazinam, Fluopicolide, Pyridinitril, Pyrifenox, Pyroxychlor, Pyroxyfur; Pyrimidinfungizide, wie Bupirimate, Cyprodinil, Diflumetorim, Dimethirimol, E-thirimol, Fenarimol, Ferimzon, Mepanipyrim, Nuarimol, Pyrimethanil, Triarimol; Pyrrolfungizide, wie Fenpiclonil, Fludioxonil, Fluoroimide; Chinolinfungizide, wie Ethoxyquin, Halacrinate, 8-Hydroxyquinolinesulfate, Chinacetol, Chinoxyfen; Chinonfungizide, wie Benquinox, Chloranil, Dichlon, Dithianon; Chinoxalinfungizide, wie Chinomethionat, Chlorquinox, Thioquinox; Thiazolfungizide, wie Ethaboxam, Etridiazol, Metsulfovax, Octhilinon, Thiabendazol, Thiadifluor, Thifluzamide; Thiocarbamatfungizide, wie Methasulfocarb, Prothiocarb; Thiophenfungizide, wie Ethaboxam, Silthiofam; Triazinfungizide, wie Anilazin; Triazolfungizide, wie Bitertanol, Fluotrimazol, Triazbutil; Harnstofffungizide, wie Bentaluron, Pencycuron, Chinazamid; unklassifzierte Fungizide, wie Acibenzolar, Acypetacs, Allylalkohol, Benzalkoniumchlorid, Benzamacril, Bethoxazin, Carvon, DBCP, Dehydroessigsäure, Diclomezine, Diethylpyrocarbonate, Fenaminosulf, Fenitropan, Fenpropidin, Formaldehyde, Furfural, Hexachlorbutadiene, Isoprothiolan, Methylisothiocyanate, Metrafenon, Nitrostyrene, Nitrothal-Isopropyl, OCH, Phthalid, Piperalin, Probenazol, Proquinazid, Pyroquilon, Natriumorthophenylphenoxid, Spiroxamine, Sultropen, Thicyofen, Tricyclazol, Zinknaphthenat.

Gemäß einer besonderen Ausführungsform der Erfindung umfassen Fungizide (a1):

Acylalanine, wie Benalaxyl, Metalaxyl, Ofurace, Oxadixyl;
Aminderivate, wie Aldimorph, Dodine, Dodemorph, Fenpropimorph, Fenpropidin, Guazatine, Iminoctadine, Spiroxamine, Tridemorph;
Anilinopyrimidine, wie Pyrimethanil, Mepanipyrimorcyprodinil;
Antibiotika, wie Cycloheximide, Griseofulvin, Kasugamycin, Natamycin, Polyoxin und Streptomycin;
Azole: Azaconazol, Bitertanol, Bromoconazol, Cyproconazol, Dichlobutrazol, Difenoconazol, Dinitroconazol, Epoxiconazol, Fenbuconazol, Fluquinconazol, Flusilazol, Flutriafol, Ketoconazol, Hexaconazol, Metconazol, Myclobutanil, Penconazol, Propiconazol, Prothioconazol, Tebuconazol, Tetraconazol, Triadimefon, Triadimenol, Triflumizol, Triticonazole;
Dicarboximide, wie Iprodion, Myclozolin, Procymidon, Vinclozolin;
Dithiocarbamate: Ferbam, Nabam, Maneb, Mancozeb, Metam, Metiram, Propineb, Polycarbamate, Thiram, Ziram, Zineb;
Heterozyklische Verbindungen, wie Anilazine, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolane, Mepronil, Nuarimol, Probenazol, Proquinazid, Pyrifenox, Pyroquilon, Chinoxyfen, Silthiofam, Thiabendazol, Thifluzamide, Thiophenat-Methyl, Tiadinil, Tricyclazol, Triforine;
Nitrophenylderivate, wie Binapacryl, Dinocap, Dinobuton, Nitrophthal-Isopropyl; Phenylpyrrole, wie Fenpiclonil und Fludioxonil;
2-Methoxybenzophenone wie in EP-A897904 beschrieben, z.B. Metrafenone;
zu keiner anderen Klasse gehörige Fungizide, wie Acibenzolar-S-Methyl, Benthiavalicarb, Carpropamid, Chlorothalonil, Cyflufenamid, Cymoxanil, Diclomezine, Diclocymet, Diethofencarb, Edifenphos, Ethaboxam, Fenhexamid, Fentin-Acetate, Fenoxanil, Ferimzon, Fluazinam, Fosetyl, Foestyl-Aluminum, Iprovalicarb, Metrafenon, Pencycuron, Propamocarb, Phthalide, Toloclofos-Methyl, Chintozene, Zoxamide;
Strobilurine wie in WO03/075663 beschrieben, z.B. Azoxystrobin, Dimoxystrobin, Fluoxastrobin, Kresoxim-Methyl, Metominostrobin, Orysastrobin, Picoxystrobin, Pyraclostrobin und Trifloxystrobin;
Sulfonate, wie Captafol, Captan, Dichlofluanid, Folpet, Tolylfluanid;
Cinnamide und ihre Analoga, wie Dimethomorph, Flumetover, Flumorph; 6-Aryl-[1,2,4]triazol[1,5-a]-pyrimidine wie z.B. in WO98/46608, WO99/41255 oder WO03/004465 beschrieben, z.B. 5-Chlor-7-(4-methylpiperidin-1-yl)-6-(2, 4, 6-trifluorphenyl)-[1,2,4]triazol[1,5-a]-pyrimidin, 5-Chlor-7-(4-methylpiperazin-1-yl)-6-(2, 4, 6-trifluorphenyl)-[1,2,4]triazol[1,5-a]-pyrimidin, 5-Chlor-7-(Morpholin-1-yl)-6-(2, 4, 6-trifluorphenyl)-[1,2,4]triazol[1,5-a]-pyrimidin, 5-Chlor-7-(Piperidin-1-yl)-6-(2, 4, 6-trifluorphenyl)-[1,2,4]triazol[1,5-a]-pyrimidin, 5-Chlor-7-(Morpholin-1-yl)-6-(2, 4, 6-trifluorphenyl)-[1,2,4]triazol[1,5-a]-pyrimidin, 5-Chlor-7-(isopropylamino)-6-(2, 4, 6-trifluorphenyl)-[1,2,4]triazol[1,5-a]-pyrimidin, 5-Chlor-7-(cyclopentylamino)-6-(2, 4, 6-trifluorphenyl)-[1,2,4]triazol[1,5-a]-pyrimidin, 5-Chlor-7-(2, 2, 2-trifluorethylamino)-6-(2, 4, 6-trifluorphenyl)-[1,2,4]triazol[1,5-a]-pyrimidin, 5-Chlor-7-(1, 1, 1-trifluorpropan-2-ylamino)-6-(2, 4, 6-trifluorphenyl)-[1,2,4]triazol[1,5-a]-pyrimidin, 5-Chlor-7-(3, 3-Dimethylbutan-2-ylamino)-6-(2, 4, 6-trifluorphenyl)-[1,2,4]triazol[1,5-a]-pyrimidin, 5-Chlor-7-(cyclohexylmethyl)-6-(2, 4, 6-trifluorphenyl)-[1,2,4]triazol[1,5-a]-pyrimidin, 5-Chlor-7-(cyclohexyl)-6-(2, 4, 6-trifluorphenyl)-[1,2,4]triazol[1,5-a]-pyrimidin, 5-Chlor-7-(2-methylbutan-3-yl)-6-(2, 4, 6-trifluorphenyl)-[1,2,4]triazol[1,5-a]-pyrimidin, 5-Chlor-7-(3-methylpropan-1-yl)-6-(2, 4, 6-trifluorphenyl)-[1,2,4]triazol[1,5-a]-pyrimidin, 5-Chlor-7-(4-methylcyclohexan-1-yl)-6-(2, 4, 6-trifluorphenyl)-[1,2,4]triazol[1,5-a]-pyrimidin, 5-Chlor-7-(Hexan-3-yl)-6-(2, 4, 6-trifluorphenyl)-[1,2,4]triazol[1,5-a]-pyrimidin, 5-Chlor-7-(2-methylbutan-1-yl)-6-(2, 4, 6-trifluorphenyl)-[1,2,4]triazol[1,5-a]-pyrimidin, 5-Chlor-7-(3-methylbutan-1-yl)-6-(2, 4, 6-trifluorphenyl)-[1,2,4]triazol[1,5-a]-pyrimidin, 5-Chlor-7-(1-methylpropan-1-yl)-6-(2, 4, 6-trifluorphenyl)-[1,2,4]triazol[1,5-a]-pyrimidin, 5-methyl-7-(4-methylpiperidin-1-yl)-6-(2, 4, 6-trifluorphenyl)-[1,2,4]triazol[1,5-a]-pyrimidin, 5-Methyl-7-(4-methylpiperazin-1-yl)-6-(2, 4, 6-trifluorphenyl)-[1,2,4]triazol[1,5-a]-pyrimidin, 5-Methyl-7-(morpholin-1-yl)-6-(2, 4, 6-trifluorphenyl)-[1,2,4]triazol[1,5-a]-pyrimidin, 5-Methyl-7-(Piperidin-1-yl)-6-(2, 4, 6-trifluorphenyl)-[1,2,4]triazol[1,5-a]-pyrimidin, 5-Methyl-7-(Morpholin-1-yl)-6-(2, 4, 6-trifluorphenyl)-[1,2,4]triazol[1,5-a]-pyrimidin, 5-Methyl-7-(Isopropylamino)-6-(2, 4, 6-trifluorphenyl)-[1,2,4]triazol[1,5-a]-pyrimidin, 5-Methyl-7-(cyclopentylamino)-6-(2, 4, 6-trifluorphenyl)-[1,2,4]triazol[1,5-a]-pyrimidin, 5-Methyl-7-(2, 2, 2-trifluorethylamino)-6-(2, 4, 6-trifluorphenyl)-[1,2,4]triazol[1,5-a]-pyrimidin, 5-Methyl-7-(1, 1, 1-trifluorpropan-2-ylamino)-6-(2, 4, 6-trifluorphenyl)-[1,2,4]triazol[1,5-a]-pyrimidin, 5-Methyl-7-(3, 3-dimethylbutan-2-ylamino)-6-(2, 4, 6-trifluorphenyl)-[1,2,4]triazol[1,5-a]-pyrimidin, 5-Methyl-7-(cyclohexylmethyl)-6-(2, 4, 6-trifluorphenyl)-[1,2,4]triazol[1,5-a]-pyrimidin, 5-Methyl-7-(cyclohexyl)-6-(2, 4, 6-trifluorphenyl)-[1,2,4]-triazol[1,5-a]-pyrimidin, 5-Methyl-7-(2-methylbutan-3-yl)-6-(2, 4, 6-trifluorphenyl)-[1,2,4]triazol[1,5-a]-pyrimidin, 5-Methyl-7-(3-methylpropan-1-yl)-6-(2, 4, 6-trifluorphenyl)-[1,2,4]triazol[1,5-a]-pyrimidin, 5-Methyl-7-(4-methylcyclohexan-1-yl)-6-(2, 4, 6-trifluorphenyl)-[1,2,4]triazol[1,5-a]-pyrimidin, 5-Methyl-7-(Hexan-3-yl)-6-(2, 4, 6-trifluorphenyl)-[1,2,4]triazol[1,5-a]-pyrimidin, 5-Methyl-7-(2-methylbutan-1-yl)-6-(2, 4, 6-trifluorphenyl)-[1,2,4]triazol[1,5-a]-pyrimidin, 5-Methyl-7-(3-methylbutan-1-yl)-6-(2, 4, 6-trifluorphenyl)-[1,2,4]triazol[1,5-a]-pyrimidine und 5-Methyl-7-(1-methylpropan-1-yl)-6-(2, 4, 6-trifluorphenyl)-[1,2,4]triazol[1,5-a]-pyrimidine;

Amidfungizide, wie Cyclofenamid, und (Z)-N-[a-(Cyclopropylmethoxyimino)-2, 3-difluor-6-(difluormethoxy)benzyl]-2-phenylacetamid.

Herbizide (a2) umfassen zum Beispiel Amidherbizide, wie Allidochlor, Beflubutamid, Benzadox, Benzipram, Bromobutide, Cafenstrole, CDEA, Chlorthiamid, Cyprazole, Dimethenamid, Dimethenamid-P, Diphenamid, Epronaz, Etnipromid, Fentrazamide, Flupoxam, Fomesafen, Halosafen, Isocarbamid, Isoxaben, Napropamide, Naptalam, Pethoxamid, Propyzamide, Chinonamid, Tebutam; insbesondere Anilidherbizide, wie Chloranocryl, Cisanilide, Clomeprop, Cypromid, Diflufenican, Etobenzanid, Fenasulam, Flufenacet, Flufenican, Mefenacet, Mefluidide, Metamifop, Monalide, Naproanilide, Pentanochlor, Picolinafen, Propanil; insbesondere Arylalaninherbizide, wie Benzoylprop, Flamprop, Flamprop-M; Chloracetanilidherbizide, wie Acetochlor, Alachlor, Butachlor, Butenachlor, Delachlor, Diethatyl, Dimethachlor, Metazachlor, Metolachlor, S-Metolachlor, Pretilachlor, Propachlor, Propisochlor, Prynachlor, Terbuchlor, Thenylchlor, Xylachlor; und Sulfonanilidherbizide, wie Benzofluor, Cloransulam, Diclosulam, Florasulam, Flumetsulam, Metosulam, Perfluidon, Pyrimisulfan, Profluazol; und Sulfonamidherbizide, wie Asulam, Carbasulam, Fenasulam, Oryzalin, Penoxsulam; Antibioticherbizide, wie Bilanafos; Aromatensäurenherbizide; insbesondere Benzoatherbizide, wie Chloramben, Dicamba, 2, 3, 6-TBA, Tricamba; insbesondere Pyrimidinyloxybenzoatherbizide, wie Bispyribac, Pyriminobac; und Pyrimidinylthiobenzoatherbizide, wie Pyrithiobac; Phthalatherbizide, wie Chlorthal; Picolinatherbizide, wie Aminopyralid, Clopyralid, Picloram; und Chinolinecarboxylatherbizide, wie Chinclorac, Chinmerac; Arsenherbizide, wie Cacodylat, CMA, DSMA, Hexaflurate, MAA, MAMA, MSMA, Kaliumarsenit, Natriumarsenit; Benzoylcyclohexandionherbizide, wie Mesotrion, Sulcotrion; Benzofuranylalkylsulfonatherbizide, wie Benfuresat, Ethofumesat; Carbamatherbizide, wie Asulam, Carboxazol, Chlorprocarb, Dichlormat, Fenasulam, Karbutilate, Terbucarb; Carbanilatherbizide, wie Barban, BCPC, Carbasulam, Carbetamid, CEPC, Chlorbufam, Chlorpropham, CPPC, Desmedipham, Phenisopham, Phenmedipham, Phenmedipham-Ethyl, Propham, Swep; Cyclohexenoximherbizide, wie Alloxydim, Butroxydim, Clethodim, Cloproxydim, Cycloxydim, Profoxydim, Sethoxydim, Tepraloxydim, Tralkoxydim; Cyclopropylisoxazolherbizide, wie Isoxachlortol, Isoxaflutol; Dicarboximidherbizide, wie Benzfendizon, Cinidon-Ethyl, Flumezin, Flumiclorac, Flumioxazin, Flumipropyn; Dinitroanilinherbizide, wie Benfluralin, Butralin, Dinitramine, Ethalfluralin, Fluchloralin, Isopropalin, Methalpropalin, Nitralin, Oryzalin, Pendimethalin, Prodiamine, Profluralin, Trifluralin; Dinitrophenolherbizide, wie Dinofenat, Dinoprop, Dinosam, Dinoseb, Dinoterb, DNOC, Etinofen, Medinoterb; Diphenyletherherbizide, wie Ethoxyfen; insbesondere Nitrophenyletherherbizide, wie Acifluorfen, Aclonifen, Bifenox, Chlomethoxyfen, Chlornitrofen, Etnipromid, Fluorodifen, Fluoroglycofen, Fluoronitrofen, Fomesafen, Furyloxyfen, Halosafen, Lactofen, Nitrofen, Nitrofluorfen, Oxyfluorfen; Dithiocarbamatherbizide, wie Dazomet, Metam; halogenaliphatische Herbizide, wie Alorac, Chloropon, Dalapon, Flupropanat, Hexachloraceton, Chloressigsäure, SMA, TCA; Imidazolinonherbizide, wie Imazamethabenz, Imazamox, Imazapic, Imazapyr, Imazaquin, Imazethapyr; anorganische Herbizide, wie Ammoniumsulfamate, Calciumchlorat, Kupfersulfat, Eisensulfat, Kaliumazid, Zyankali, Natriumazid, Natriumchlorat, Schwefelsäure; Nitrilherbizide, wie Bromobonil, Bromo-

xynil, Chloroxynil, Dichlobenil, Iodobonil, Ioxynil, Pyraclonil; Organophosphorherbizide, wie Amiprofos-Methyl, Anilofos, Bensulide, Bilanafos, Butamifos, 2,4-DEP, DMPA, EBEP, Fosamin, Glufosinat, Glyphosat, Piperophos; Phenoxyherbizide, wie Bromofenoxim, Clomeprop, 2,4-DEB, 2,4-DEP, Difenopenten, Disul, Erbon, Etnipromid, Fenteracol, Trifopsime; insbesondere Phenoxyessigsäureherbizide, wie 4-CPA, 2,4-D, 3,4-DA, MCPA, MCPA-Thioethyl; Phenoxybuttersäureherbizide, wie 4-CPB, 2,4-DB, 3,4-DB, MCPB, 2,4,5-TB; und Phenoxypropionsäureherbizide, wie Cloprop, 4-CPP, Dichlorprop, Dichlorprop-P, 3, 4-DP, Fenoprop, Mecoprop, Mecoprop-P; insbesondere Aryloxyphenoxypropionsäureherbizide, wie Chlorazifop, Clodinafop, Clofop, Cyhalofop, Diclofop, Fenoxaprop, Fenoxaprop-P, Fenthiaprop, Fluazifop, Fluazifop-P, Haloxyfop, Haloxyfop-P, Isoxapyrifop, Metamifop, Propaquizafop, Quizalofop, Quizalofop-P, Trifop; Phenylenediaminherbizide, wie Dinitramine, Prodiamine; Phenylpyrazolylketonherbizide, wie Benzofenap, Pyrazolynate, Pyrazoxyfen, Topramezone; Pyrazolylphenylherbizide, wie Fluazolate, Pyraflufen; Pyridazinherbizide, wie Credazin, Pyridafol, Pyridat; Pyridazinonherbizide, wie Brompyrazon, Chloridazon, Dimidazon, Flufenpyr, Metflurazon, Norflurazon, Oxapyrazon, Pydanon; Pyridinherbizide, wie Aminopyralid, Cliodinate, Clopyralid, Dithiopyr, Fluroxypyr, Haloxydine, Picloram, Picolinafen, Pyriclor, Thiazopyr, Triclopyr; Pyrimidinediaminherbizide, wie Iprymidam, Tioclorim; quarternäre Ammoniumherbizide, wie Cyperquat, Diethamquat, Difenzoquat, Diquat, Morfamquat, Paraquat; Thiocarbamatherbizide, wie Butylate, Cycloate, Di-Allate, EPTC, Esprocarb, Ethiolate, Isopolinate, Methiobencarb, Molinate, Orbencarb, Pebulate, Prosulfocarb, Pyributicarb, Sulfallate, Thiobencarb, Tiocarbazil, TriAllate, Vernolate; Thiocarbonatherbizide, wie Dimexano, EXD, Proxan; Thioharnstoffherbizide, wie Methiuron; Triazinherbizide, wie Dipropetryn, Triaziflam, Trihydroxytriazine; insbesondere Chlortriazinherbizide, wie Atrazine, Chlorazine, Cyanazine, Cyprazine, Eglinazine, Ipazine, Mesoprazine, Procyazine, Proglinazine, Propazine, Sebuthylazine, Simazine, Terbuthylazine, Trietazine; Methoxytriazinherbizide, wie Atraton, Methometon, Prometon, Secbumeton, Simeton, Terbumeton; und Methylthiotriazinherbizide, wie Ametryn, Aziprotryne, Cyanatryn, Desmetryn, Dimethametryn, Methoprotryne, Prometryn, Simetryn, Terbutryn; Triazinonherbizide, wie Ametridion, Amibuzin, Hexazinon, Isomethiozin, Metamitron, Metribuzin; Triazolherbizide, wie Amitrole, Cafenstrol, Epronaz, Flupoxam; Triazolonherbizide, wie Amicarbazon, Carfentrazon, Flucarbazon, Propoxycarbazon, Sulfentrazone; Triazolopyrimidinherbizide, wie Cloransulam, Diclosulam, Florasulam, Flumetsulam, Metosulam, Penoxsulam; Uracilherbizide, wie Butafenacil, Bromacil, Flupropacil, Isocil, Lenacil, Terbacil; Harnstoffherbizide, wie Benzthiazuron, Cumyluron, Cycluron, Dichloralharnstoff, Diflufenzopyr, Isonoruron, Isouron, Methabenzthiazuron, Monisouron, Noruron; insbesondere Phenylharnstoffherbizide, wie Anisuron, Buturon, Chlorbromuron, Chloreturon, Chlorotoluron, Chloroxuron, Daimuron, Difenoxuron, Dimefuron, Diuron, Fenuron, Fluometuron, Fluothiuron, Isoproturon, Linuron, Methiuron, Methyldymron, Metobenzuron, Metobromuron, Metoxuron, Monolinuron, Monuron, Neburon, Parafluron, Phenobenzuron, Siduron, Tetrafluron, Thidiazuron; Sulfonylharnstoffherbizide; insbesondere Pyrimidinylsulfonylharnstoffherbizide, wie Amidosulfuron, Azimsulfuron, Bensulfuron, Chlorimuron, Cyclosulfamuron, Ethoxysulfuron, Flazasulfuron, Flucetosulfuron, Flupyrsulfuron, Foramsulfuron, Halosulfuron, Imazosulfuron, Mesosulfuron, Nicosulfuron, Orthosulfamuron, Oxasulfuron, Primisulfuron, Pyrazosulfuron, Rimsulfuron, Sulfometuron, Sulfosulfuron, Trifloxysulfuron; und Triazinylsulfonylharnstoffherbizide, wie Chlorsulfuron, Cinosulfuron, Ethametsulfuron, Iodosulfuron, Metsulfuron, Prosulfuron, Thifensulfuron, Triasulfuron, Tribenuron, Triflusulfuron, Tritosulfuron; und Thiadiazolylharnstoffherbizide, wie Buthiuron, Ethidimuron, Tebuthiuron, Thiazafluron, Thidiazuron; und sonstige Herbizide, wie Acrolein, Allylalkohol, Azafenidin, Benazolin, Bentazon, Benzobicyclon, Buthidazole, Calciumcyanamid, Cambendichlor, Chlorfenac, Chlorfenprop, Chlorflurazole, Chlorflurenol, Cinmethylin, Clomazon, CPMF, Cresol, Orthodichlorbenzol, Dimepiperate, Endothal, Fluoromidine, Fluridon, Flurochloridon, Flurtamon, Fluthiacet, Indanofan, Methazole, Methylisothiocyanate, Nipyraclofen, OCH, Oxadiargyl, Oxadiazon, Oxaziclomefon, Pentoxazon, Pinoxaden, Prosulfalin, Pyribenzoxim, Pyriftalid, Chinoclamine, Rhodethanil, Sulglycapin, Thidiazimin, Tridiphane, Trimeturon, Tripropindan, Tritac.

Gemäß einer besonderen Ausführungsform der Erfindung umfassen Herbizide (a2):

Lipid-Biosynthese-Inhibitoren wie beispielsweise Chlorazifop, Clodinafop, Clofop, Cyhalofop, Diclofop, Fenoxaprop, Fenoxaprop-p, Fenthiaprop, Fluazifop, Fluazifop-P, Haloxyfop, Haloxyfop-P, Isoxapyrifop, Metamifop, Propaquizafop, Quizalofop, Quizalofop-P, Trifop, Alloxydim, Butroxydim, Clethodim, Cloproxydim, Cycloxydim, Profoxydim, Sethoxydim, Tepraloxydim, Tralkoxydim, Butylat, Cycloat, Diallat, Dimepiperat, EPTC, Esprocarb, Ethiolat, Isopolinat, Methiobencarb, Molinat, Orbencarb, Pebulat, Prosulfocarb, Sulfallat, Thiobencarb, Tiocarbazil, Triallate, Vernolat, Benfuresat, Ethofumesat und Bensulid;

ALS-Inhibitoren wie beispielsweise Amidosulfuron, Azimsulfuron, Bensulfuron, Chlorimuron, Chlorsulfuron, Cinosulfuron, Cyclosulfamuron, Ethametsulfuron, Ethoxysulfuron, Flazasulfuron, Flupyrsulfuron, Foramsulfuron, Halosulfuron, Imazosulfuron, Iodosulfuron, Mesosulfuron, Metsulfuron, Nicosulfuron, Oxasulfuron, Primisulfuron, Prosulfuron, Pyrazosulfuron, Rimsulfuron, Sulfometuron, Sulfosulfuron, Thifensulfuron, Triasulfuron, Tribenuron, Trifloxysulfuron, Triflusulfuron, Tritosulfuron, Imazamethabenz, Imazamox, Imazapic, Imazapyr, Imazaquin, Imazethapyr, Cloransulam, Diclosulam, Florasulam, Flumetsulam, Metosulam, Penoxsulam, Bispyribac, Pyriminobac, Propoxycarbazone, Flucarbazone, Pyribenzoxim, Pyriftalid und Pyrithiobac; Photosynthese-Inhibitoren wie beispielsweise Atraton, Atrazin, Ametryn, Aziprotryn, Cyanazin, Cyanatryn, Chlorazin, Cyprazin, Desmetryn, Dimethametryn, Dipropetryn, Eglinazin, Ipazin, Mesoprazin, Methometon, Methoprotryn, Procyazin, Proglinazin, Prometon, Prometryn, Propazin, Sebuthylazin, Secbumeton, Simazin, Simeton, Simetryn, Terbumeton, Terbuthylazine, Tterbutryn, Trietazin, Ametridion, Amibuzin, Hexazinon, Isomethiozin, Metamitron, Metribuzin, Bromacil, Isocil, Lenacil, Terbacil, Brompyrazon, Chloridazon, Dimidazon, Desmedipham, Phenisopham, Phenmedipham, Phenmedipham-Ethyl, Benzthiazuron, Buthiuron, Ethidimuron, Isouron, Methabenzthiazuron, Monoisouron, Tebuthiuron, Thiazafluron, Anisuron, Buturon, Chlorbromuron, Chloreturon, Chlorotoluron, Chloroxuron, Difenoxuron, Dimefuron, Diuron, Fenuron, Fluometuron, Fluothiuron, Isoproturon, Linuron, Methiuron, Metobenzuron, Metobromuron, Metoxuron, Monolinuron, Monuron, Neburon, Parafluron, Phenobenzuron, Siduron, Tetrafluron, Thidiazuron, Cyperquat, Diethamquat, Difenzoquat, Diquat, M;orfamquat, Paraquat, Bromobonil, Bromoxynil, Chloroxynil, lodobonil, loxynil, Amicarbazon, Bromofenoxim, Flumezin, Methazol, Bentazon, Propanil, Pentanochlor, Pyridate und Pyridafol;
Protoporphyrinogen-IX-Oxidase-Inhibitoren wie beispielsweise Acifluorfen, Bifenox, Chlomethoxyfen, Chlornitrofen, Ethoxyfen, Fluorodifen, Fluoroglycofen, Fluoronitrofen, Fomesafen, Furyloxyfen, Halosafen, Lactofen, Nitrofen, Nitrofluorfen, Oxyfluorfen, Fluazolate, Pyraflufen, Cinidonethyl, Flumiclorac, Flumioxazin, Flumipropyn, Fluthiacet, Thidiazimin, Oxadiazon, Oxadiargyl, Azafenidin, Carfentrazon, Sulfentrazon, Pentoxazon, Benzfendizon, Butafenacil, Pyraclonil, Profluazol, Flufenpyr, Flupropacil, Nipyraclofen und Etnipromid;
Bleacher-Herbizide wie beispielsweise Metflurazon, Norflurazon, Flufenican, Diflufenican, Picolinafen, Beflubutamid, Fluridon, Flurochloridon, Flurtamon, Mesotrion, Sulcotrion, Isoxachlortol, Isoxaflutol, Benzofenap, Pyrazolynat, Pyrazoxyfen, Benzobicyclon, Amitrole, Clomazon, Aclonifen, 4-(3-Trifluormethylphenoxy)- 2-(4-trifluormethylphenyl)pyrimidin sowie 3-Heterocyclyl-substituierte Benzoylderivative der Formel II (siehe WO 96/26202, WO 97/41116, WO 97/41117 und WO 97/41118)

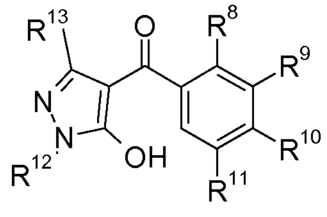

(II)

in der die Variablen $R^8$ bis $R^{13}$ die folgenden Bedeutungen haben:

- $R^8$, $R^{10}$ Wasserstoff, Halogen, $C_1$-$C_6$-Alkyl, $C_1$-$C_6$-Haloalkyl, $C_1$-$C_6$-Alkoxy, $C_1$-$C_6$-Haloalkoxy, $C_1$-$C_6$-Alkylthio, $C_1$-$C_6$-Alkylsulfinyl oder $C_1$-$C_6$-Alkylsulfonyl;
- $R^9$ ein heterocyclischer Rest ausgewählt aus der Gruppe Thiazol-2-yl, Thiazol-4-yl, Thiazol-5-yl, Isoxazol-3-yl, Isoxazol-4-yl, Isoxazol-5-yl, 4,5-Dihydroisoxazol-3-yl, 4,5-Dihydroisoxazol-4-yl und 4,5-Dihydroisoxazol-5-yl, wobei die neun genannten Reste unsubstituiert oder mono- oder polysubstituiert sein können, beispielsweise mono-, di-, tri- oder tetra-substituiert durch Halogen, $C_1$-$C_4$-Alkyl, $C_1$-$C_4$-Alkoxy, $C_1$-$C_4$-Haloalkyl, $C_1$-$C_4$-Haloalkoxy oder $C_1$-$C_4$-Alkylthio;
- $R^{11}$ Wasserstoff, Halogen or $C_1$-$C_6$-Alkyl;
- $R^{12}$ $C_1$-$C_6$-Alkyl;
- $R^{13}$ Wasserstoff oder $C_1$-$C_6$-Alkyl.

EPSP-Synthase-Inhibitoren wie beispielsweise Glyphosat;
Glutamin-Synthase-Inhibitoren wie beispielsweise Glufosinat und Bilanaphos;
DHP-Synthase-Inhibitoren wie beispielsweise Asulam;
Mitose-Inhibitoren wie beispielsweise Benfluralin, Butralin, Dinitramin, Ethalfluralin, Fluchloralin, Isopropalin, Methalpropalin, Nitralin, Oryzalin, Pendimethalin, Prodiamin, Profluralin, Trifluralin, Amiprofosmethyl, Butamifos, Dithiopyr, Thiazopyr, Propyzamid, Tebutam, Chlorthal, Carbetamid, Chlorbufam, Chlorpropham und Propham; VLCFA-Inhibitoren wie beispielsweise Acetochlor, Alachlor, Butachlor, Butena-chlor, Delachlor, Diethatyl, Dimethachlor, Dimethenamid, Dimethenamid-P, Metaza-chlor, Metolachlor, S-Metolachlor, Pretilachlor, Propachlor, Propisochlor, Prynachlor, Terbuchlor, Thenylchlor, Xylachlor, Allidochlor, CDEA, Epronaz, Diphenamid, Napropamide, Naproanilide, Pethoxamid, Flufenacet, Mefenacet, Fentrazamid, Anilofos, Piperophos, Cafenstrol, Indanofan und Tridiphan;
Cellulose-Biosynthese-Inhibitoren wie beispielsweise Dichlobenil, Chlorthiamid, Isoxaben und Flupoxam;
Entkoppler-Herbizide wie beispielsweise Dinofenat, Dinoprop, Dinosam, Dinoseb, Dinoterb, DNOC, Etinofen und Medinoterb;
Auxin-Herbizide wie beispielsweise Clomeprop, 2,4-D, 2,4,5-T, MCPA, MCPA Thioethyl, Dichlorprop, Dichlorprop-P, Mecoprop, Mecoprop-P, 2,4-DB, MCPB, Chloramben, Dicamba, 2,3,6-TBA, Tricamba, Quinclorac, Quinmerac, Clopyralid, Fluroxypyr, Picloram, Triclopyr und Benazolin;

Auxin-Transport-Inhibitoren wie beispielsweise Naptalam und Diflufenzopyr; und Benzoylprop, Flamprop, Flamprop-M, Bromobutide, Chlorflurenol, Cinmethylin, Methyldymron, Etobenzanid, Fosamine, Metam, Pyributicarb, Oxaziclomefone, Dazomet, Triaziflam und Methylbromid.

Insektizide (a3) umfassen zum Beispiel Antibiotika-Insektizide, wie Allosamidin, Thuringiensin; insbesondere macrocyclische Lactoninsektizide, wie Spinosad; insbesondere Vermectininsektizide, wie Abamectin, Doramectin, Emamectin, Eprinomectin, Ivermectin, Selamectin; und Milbemycininsektizide, wie Lepimectin, Milbemectin, Milbemycinoxime, Moxidectin; Arseninsektizide, wie Calciumarsenat, Kupferacetarsenit, Kupferarsenat, Bleiarsenat, Kaliumarsenit, Natriumarsenit; pflanzliche Insektizide, wie Anabasin, Azadirachtin, D-Limonen, Nicotin, Pyrethrine, Cinerin E, Cinerin I, Cinerin II, Jasmolin I, Jasmolin II, Pyrethrin I, Pyrethrin II, Quassia, Rotenon, Ryania, Sabadilla; Carbamatinsektizide, wie Bendiocarb, Carbaryl; insbesondere Benzofuranylmethylcarbamatinsektizide, wie Benfuracarb, Carbofuran, Carbosulfan, Decarbofuran, Furathiocarb; Dimethylcarbamatinsektizide, wie Dimetan, Dimetilan, Hyquincarb, Pirimicarb; Oximcarbamatinsektizide, wie Alanycarb, Aldicarb, Aldoxycarb, Butocarboxim, Butoxycarboxim, Methomyl, Nitrilacarb, Oxamyl, Tazimcarb, Thiocarboxime, Thiodicarb, Thiofanox; und Phenylmethylcarbamatinsektizide, wie Allyxycarb, Aminocarb, Bufencarb, Butacarb, Carbanolate, Cloethocarb, Dicresyl, Dioxacarb, EMPC, Ethiofencarb, Fenethacarb, Fenobucarb, Isoprocarb, Methiocarb, Metolcarb, Mexacarbate, Promacyl, Promecarb, Propoxur, Trimethacarb, XMC, Xylylcarb; Dinitrophenolinsektizide, wie Dinex, Dinoprop, Dinosam, DNOC; Insektenwachstumsregulatoren; insbesondere Chitinsyntheseinhibitoren, wie Bistrifluron, Buprofezin, Chlorfluazuron, Cyromazine, Diflubenzuron, Flucycloxuron, Flufenoxuron, Hexaflumuron, Lufenuron, Novaluron, Noviflumuron, Penfluron, Teflubenzuron, Triflumuron; Juvenilhormonmimetika, wie Epofenonan, Fenoxycarb, Hydropren, Kinopren, Methopren, Pyriproxyfen, Tripren; Juvenilhormone, wie Juvenilhormon I, II und III; Häutungshormonagonisten, wie Chromafenozid, Halofenozid, Methoxyfenozid, Tebufenozid; Häutungshormone, wie A-Ecdyson, Ecdysteron; Häutungshemmer, wie Diofenolan; Precocene, wie Precocen I, II und III; und unklassifizierte Insektizide, wie Dicyclanil; Nereistoxinanaloga, wie Bensultap, Cartap, Thiocyclam, Thiosultap; Nicotinoidinsektizide, wie Flonicamid; insbesondere Nitroguanidininsektizide, wie Clothianidin, Dinotefuran, Imidacloprid, Thiamethoxam; Nitromethyleninsektizide, wie Nitenpyram, Nithiazine; und Pyridylmethylamininsektizide, wie Acetamiprid, Imidacloprid, Nitenpyram, Thiacloprid; Organochlorininsektizide, wie Isobenzan, Isodrin, Kelevan, Mirex; Organophosphorinsektizide; insbesondere Organophosphatinsektizide, wie Bromfenvinfos, Chlorfenvinphos, Crotoxyphos, Dichlorvos, Dicrotophos, Dimethylvinphos, Fospirate, Heptenophos, Methocrotophos, Mevinphos, Monocrotophos, Naled, Naftalofos, Phosphamidon, Propaphos, TEPP, Tetrachlorvinphos; Organothiophosphatinsektizide, wie Dioxabenzofos, Fosmethilan, Phenthoate; insbesondere aliphatische Organothiophosphatinsektizide, wie Acethion, Amiton, Cadusafos, Chlorethoxyfos, Chlormephos, Demephion, Demephion-O, Demephion-S, Demeton, Demeton-O, Demeton-S, Demeton-Methyl, Demeton-O-Methyl, Demeton-S-Methyl, Demeton-S-Methylsulphon, Disulfoton, Ethion, Ethoprophos, IPSP, Isothioat, Malathion, Methacrifos, Oxydemeton-Methyl, Oxydeprofos, Oxydisulfoton, Phorat, Sulfotep, Terbufos, Thiometon; insbesondere aliphatische Amidorganothiophosphatinsektizide, wie Amidithion, Cyanthoate, Dimethoate, Ethoat-Methyl, Formothion, Mecarbam, Omethoate, Prothoate, Sophamide, Vamidothion; und Oximorganothiophosphatinsektizide, wie Chlorphoxim, Phoxim, Phoxim-Methyl; Heterocyclische Organothiophosphatinsektizide, wie Azamethiphos, Coumaphos, Coumithoate, Dioxathion, Endothion, Menazon, Morphothion, Phosalon, Pyraclofos, Pyridaphenthion, Chinothion; besonders Benzothiopyranorganothiophosphatinsektizide, wie Dithicrofos, Thicrofos; Benzotriazinorganothiophosphatinsektizide, wie Azinphos-Ethyl, Azinphos-Methyl; Isoindolorganothiophosphatinsektizide, wie Dialifos, Phosmet; Isoxazolorganothiophosphatinsektizide, wie Isoxathion, Zolaprofos; Pyrazolopyrimidinorganothiophosphatinsektizide, wie Chlorprazophos, Pyrazophos; Pyridinorganothiophosphatinsektizide, wie Chlorpyrifos, Chlorpyrifos-Methyl; Pyrimidinorganothiophosphatinsektizide, wie Butathiofos, Diazinon, Etrimfos, Lirimfos, Pirimiphos-Ethyl, Pirimiphos-Methyl, Primidophos, Pyrimitate, Tebupirimfos; Chinoxalinorganothiophosphatinsektizide, wie Chinalphos, Chinalphos-Methyl; Thiadiazolorganothiophosphatinsektizide, wie Athidathion, Lythidathion, Methidathion, Prothidathion; und Triazoleorganothiophosphatinsektizide, wie Isazofos, Triazophos; und Phenylorganothiophosphatinsektizide, wie Azothoate, Bromophos, Bromophos-Ethyl, Carbophenothion, Chlorthiophos, Cyanophos, Cythioate, Dicapthon, Dichlofenthion, Etaphos, Famphur, Fenchlorphos, Fenitrothion, Fensulfothion, Fenthion, Fenthion-Ethyl, Heterophos, Jodfenphos, Mesulfenfos, Parathion, Parathion-Methyl, Phenkapton, Phosnichlor, Profenofos, Prothiofos, Sulprofos, Temephos, Trichlormetaphos-3, Trifenofos; Phosphonatinsektizide, wie Butonat, Trichlorfon; Phosphonothioatinsektizide, wie Mecarphon; insbesondere Phenylethylphosphonothioatinsektizide, wie Fonofos, Trichloronat; und Phenylphenylphosphonothioatinsektizide, wie Cyanofenphos, EPN, Leptophos; Phosphoramidatinsektizide, wie Crufomate, Fenamiphos, Fosthietan, Mephosfolan, Phosfolan, Pirimetaphos; Phosphoramidothioatinsektizide, wie Acephate, Isocarbophos, Isofenphos, Methamidophos, Propetamphos; und Phosphorodiamidinsektizide, wie Dimefox, Mazidox, Mipafox, Schradan; Oxadiazininsektizide, wie Indoxacarb; Phthalimidinsektizide, wie Dialifos, Phosmet, Tetramethrin; Pyrazolinsektizide, wie Acetoprol, Ethiprol, Fipronil, Pyrafluprol, Pyriprol, Tebufenpyrad, Tolfenpyrad, Vaniliprole; Pyrethroidinsektizide; insbesondere Pyrethroidesterinsektizide, wie Acrinathrin, Allethrin, Bioallethrin, Barthrin, Bifenthrin, Bioethanomethrin, Cyclethrin, Cycloprothrin, Cyfluthrin, Beta-Cyfluthrin, Cyhalothrin, Gamma-Cyhalothrin, Lambda-Cyhalothrin, Cypermethrin, Alpha-Cypermethrin, Beta-Cypermethrin, Theta-Cypermethrin, Zeta-Cypermethrin, Cyphenothrin, Deltamethrin, Dimefluthrin, Dimethrin, Empenthrin, Fenfluthrin, Fenpirithrin, Fenpropathrin, Fenvalerate, Esfenvalerate, Flucythrinate, Fluvalinate,

Tau-Fluvalinate, Furethrin, Imiprothrin, Metofluthrin, Permethrin, Biopermethrin, Transpermethrin, Phenothrin, Prallethrin, Profluthrin, Pyresmethrin, Resmethrin, Bioresmethrin, Cismethrin, Tefluthrin, Terallethrin, Tetramethrin, Tralomethrin, Transfluthrin; und Pyrethroidetherinsektizide, wie Etofenprox, Flufenprox, Halfenprox, Protrifenbute, Silafluofen; Pyrimidinamininsektizide, wie Flufenerim, Pyrimidifen; Pyrrolinsektizide, wie Chlorfenapyr; Tetronicsäurinsektizide, wie Spiromesifen; Thioharnstoffinsektizide, wie Diafenthiuron; Harnstoffinsektizide, wie Flucofuron, Sulcofuron; unklassifizierte Insektizide, wie Closantel, Crotamiton, EXD, Fenazaflor, Fenoxacrim, Flubendiamide, Hydramethylnon, Isoprothiolane, Malonoben, Metaflumizon, Metoxadiazon, Nifluridide, Pyridaben, Pyridalyl, Rafoxanide, Triarathene, Triazamate.

Gemäß einer besonderen Ausführungsform der vorliegenden Erfindung umfassen Insektizide (a3):

Organophosphate, wie Azinphos-Methyl, Azinphos-Ethyl, Chlorpyrifos, Chlorpyrifos-Methyl, Chlorfenvinphos, Diazinon, Dimethylvinphos, Dioxabenzofos, Disulfoton, Ethion, EPN, Fenitrothion, Fenthion, Heptenophos, Isoxathion, Malathion, Methidathion, Methyl-Parathion, Paraoxon, Parathion, Phenthoate, Phosalon, Phosmet, Phorate, Phoxim, Pirimiphos-Methyl, Profenofos, Prothiofos, Primiphos-Ethyl, Pyraclofos, Pyridaphenthion, Sulprofos, Triazophos, Trichlorfon, Tetrachlorvinphos, Vamidothion;
Carbamate, wie Alanycarb, Benfuracarb, Bendiocarb, Carbaryl, Carbofuran, Carbosulfan, Fenoxycarb, Furathiocarb, Indoxacarb, Methiocarb, Pirimicarb, Propoxur, Thiodicarb, Triazamate;
Pyrethroide, wie Bifenthrin, Cyfluthrin, Cycloprothrin, Cypermethrin, Deltamethrin, Esfenvalerat, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, Alpha-Cypermethrin, Permethrin;
Arthropodenwachstumsregulatoren:

Chitinsyntheseinhibitoren, z. B. Benzoylharnstoffe, wie Chlorfluazuron, Diflubenzuron, Flucycloxuron, Flufenoxuron, Hexaflumuron, Lufenuron, Novaluron, Teflubenzuron,
Triflumuron; Buprofezin, Diofenolan, Hexythiazox, Etoxazol, Clofentazine;
Ecdysonantagonisten, wie Halofenozid, Methoxyfenozid, Tebufenozid;
Juvenoide, wie Pyriproxyfen, Methoprene;
Lipidbiosyntheseinhibitoren, wie Spirodiclofen;
Neonicotinoide, wie Flonicamid, Clothianidin, Dinotefuran, Imidacloprid, Thiamethoxam, Nithiazine, Acetamiprid, Thiacloprid;
Weitere Insektizide, die keiner der genannten Klassen zuzurechnen sind, wie Abamectin, Acequinocyl, Acetamiprid, Azadirachtin, Bensultap, Bifenazate, Cartap, Chlorfenapyr, Diafenthiuron, Dinetofuran, Diofenolan, Emamectin, Ethiprol, Fenazaquin, Fipronil, Hydramethylnon, Imidacloprid, Indoxacarb, Isoprocarb, Metolcarb, Pyridaben, Pymetrozine, Spinosad, Tebufenpyrad, Thiamethoxam, Xmc und Xylylcarb und
N-Phenylsemicarbazone wie in EP-A462456 beschrieben, insbesondere Verbindungen der allgemeinen Formel (IV)

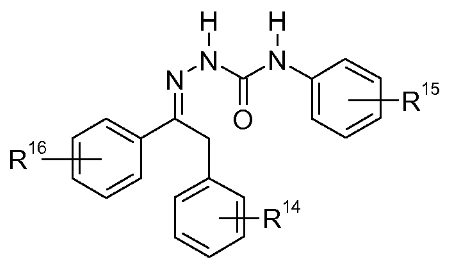

(IV),

worin $R^{14}$ und $R^{15}$ unabhängig voneinander Wasserstoff, Halogen, CN, $C_1$-$C_4$-alkyl, $C_1$-$C_4$-Alkoxy, $C_{1-4}$-Haloalkyl or $C_{1-4}$-Haloalkoxy und $R^{16}$ is $C_{1-4}$-Alkoxy, $C_{1-4}$-Haloalkyl or $C_{1-4}$-Haloalkoxy sein können, z.B. Verbindungen gemäß Formel (IV), in denen $R^{16}$ = 3-$CF_3$, $R^{14}$ = 4-CN und $R^{15}$ = 4-$OCF_3$ sind.

Von den hier speziell genannten Wirkstoffen können auch Salze, insbesondere landwirtschaftlich nutzbare Salze eingesetzt werden.

In einer besonderen Ausführungsform der Erfindung ist der Pflanzenschutzwirkstoff ein Fungizid.

Besonders bevorzugt ist es hierbei, wenn das Fungizid ein Wirkstoff aus der Gruppe der Anilide, Triazolopyrimidine, Strobilurine oder Triazole ist, insbesondere ein Anilid ausgewählt unter Boscalid, Carboxin, Metalaxyl und Oxadixyl, das Triazolopyrimidin 5-Chlor-7-(4-methylpiperidin-1-yl)-6-(2,4,6-trifluorphenyl)-[1,2,4]triazol[1,5-a]-pyrimidin, ein Strobilurin ausgewählt unter Azoxystrobin, Pyraclostrobin, Dimoxystrobin, Trifloxystrobin, Fluoxystrobin, Picoxystrobin

und Orysastrobin oder ein Triazol ausgewählt unter Epoxiconazol, Metconazol, Tebuconazol, Flusilazol, Fluquinconazol, Triticonazol, Propiconazol, Penconazol, Cyproconazol und Prothioconazol.

Erfindungsgemäß besonders bevorzugt ist Epoxiconazol.

Die hier gewählten Namen von Pflanzenschutzwirkstoffen, z.B. Epoxiconazol, schließen isomere Formen dieser Verbindung mit ein. Insbesondere zu nennen sind Stereoisomere, wie Enantiomere oder Diastereoisomere der Formeln. Neben den im Wesentlichen reinen Isomeren gehören zu den Verbindungen der Formeln auch deren Isomerengemische, z. B. Stereoisomerengemische.

Allgemein bevorzugt werden Wirkstoffe mit einem höheren Anteil des gegenüber dem optischen Antipoden biologisch wirksameren Stereoisomers, besonders bevorzugt isomerenreine Wirkstoffe.

Die vorliegende Erfindung betrifft insbesondere Mittel mit hohen Wirkstoffanteilen (Konzentrate). So macht der Anteil der Komponente (a) am Gesamtgewicht des Mittels in der Regel mehr als 5 Gew.-%, vorzugsweise mehr als 10 Gew.-% und insbesondere mehr als 20 Gew.-% aus.

Um einen ausreichenden adjuvanten Effekt zu gewährleisten, beträgt das Gewichtsverhältnis von Komponente (b) zu Komponente (a) mehr als 1 und vorteilhafterweise mehr als 2.

Die erfindungsgemäßen Mittel können im Übrigen übliche Hilfs- und/oder Zusatzstoffe für die Herstellung von Formulierungen im agrochemischen Bereich und insbesondere auf dem Gebiet des Pflanzenschutzes enthalten. Dazu gehören beispielsweise Tenside, Dispergierhilfsmittel, Netzmittel, Verdickungsmittel, organische Lösungsmittel, Cosolventien, Entschäumer, Carbonsäuren, Konservierungsmittel, Stabilisierungsmittel, etc.

Gemäß einer besonderen Ausführungsform der vorliegenden Erfindung umfassen die Mittel als oberflächenaktive Komponente (c) wenigstens ein (weiteres) Tensid. Der Begriff "Tensid" bezeichnet hier grenzflächenaktive bzw. oberflächenaktive Mittel.

Die Komponente (c) wird insbesondere als Dispergator bzw. Emulgator, vor allem zum Dispergieren eines Feststoffanteils in Suspensionskonzentraten, zugesetzt. Ferner können Teile der Komponente (c) als Netzmittel dienen.

Prinzipiell brauchbar sind anionische, kationische, amphotere und nichtionische Tenside, wobei Polymer-Tenside sowie Tenside mit Heteroatomen in der hydrophoben Gruppe eingeschlossen sind.

Zu den anionischen Tensiden gehören beispielsweise Carboxylate, insbesondere Alkali-, Erdalkali- und Ammoniumsalze von Fettsäuren, z.B. Kaliumstearat, die üblicherweise auch als Seifen bezeichnet werden; Acylglutamate; Sarkosinate, z.B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalin- und Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyl und Harnstoff, Mono- oder Dialkyl-bernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide.

Zu den nichtionischen Tensiden gehören beispielsweise weitere Alkoxylate und vor allem Ethoxylate sowie nichtionische Tenside, insbesondere

- Fettalkohol-polyoxyethylen-ester, beispielsweise Laurylalkoholpolyoxyethylenetheracetat,
- Alkyl-Polyoxyethylen- und -polyoxypropylen-ether, z.B. von Fettalkoholen mit 8 oder mehr Kohlenstoffatomen, Alkylarylalkohol-Polyoxyethylenether, z.B. Octylphenol-Polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonyl-phenol, Tributylphenol-polyoxyethylenether,
- Fettaminalkoxylate, Fettsäureamid- und Fettsäurediethanol-amidalkoxylate, insbesondere deren Ethoxylate,
- Zuckertenside, Sorbitester, wie beispielsweise Sorbitanfettsäureester (Sorbitanmonooleat, Sorbitantristearat), Polyoxy-ethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z.B. Tetradecyldimethylaminoxid.

Zu den polymeren Tensiden gehören beispielsweise Di-, Tri- und Multiblockpolymere vom Typ $(AB)_x$, ABA und BAB, z.B. gegebenenfalls Endgruppen-verschlossene Ethylenoxid/Propylenoxid-Blockcopolymere, z.B. Ethylendiamin-EO/PO-Blockcopolymere, Polystyrol-Block-Polyethylenoxid, und AB-Kammpolymere, z.B. Polymethacrylat-comb-Polyethylenoxid.

Weitere Tenside, die hier beispielhaft genannt werden sollen, sind Perfluortenside, Silikontenside, z.B. Polyether-modifizierte Siloxane, Phospholipide, wie beispielsweise Lecithin oder chemisch modifizierte Lecithine, Aminosäuretenside, z.B. N-Lauroylglutamat und oberflächenaktive Homo- und Copolymere, z.B. Polyvinylpyrrolidon, Polyacrylsäuren in Form ihrer Salze, Polyvinylalkohol, Polypropylenoxid, Polyethylenoxid, Maleinsäureanhydrid-Isobuten-Copolymere und Vinylpyrrolidon-VinylacetatCopolymere.

Sofern nicht spezifiziert, handelt es sich bei den Alkylketten der oben aufgeführten Tenside um lineare oder verzweigte Reste mit üblicherweise 8 bis 20 Kohlenstoffatomen.

Vorzugsweise ist das weitere Tensid im Rahmen der Komponente (c) ausgewählt unter nichtionischen Tensiden. Hiervon sind insbesondere diejenigen bevorzugt, die HLB-Werte im Bereich von 2 bis 16, vorzugsweise im Bereich von 5 bis 16, und insbesondere im Bereich von 8 bis 16 besitzen.

Der Anteil der Komponente (c) am Gesamtgewicht des Mittels beträgt - sofern vorhanden - in der Regel weniger als 50 Gew.-%, vorzugsweise weniger als 15 Gew.-% und insbesondere weniger als 5 Gew.-%.

Gemäß einer besonderen Ausführungsform der vorliegenden Erfindung umfassen die Mittel als Komponente (d) mindestens einen weiteren Hilfsstoff.

Die Komponente (d) kann vielerlei Zwecke erfüllen. Die Wahl geeigneter Hilfsstoffe erfolgt den Anforderungen entsprechend in üblicher Weise durch den Fachmann.

Beispielsweise sind weitere Hilfsstoffe ausgewählt unter

(d1) Lösungs- oder Verdünnungsmitteln;

(d2) Retentionsmitteln, pH-Puffern, Anti-Schaumstoffen.

Neben Wasser können die Mittel weitere Lösungsmittel löslicher Bestandteile bzw. Verdünnungsmittel unlöslicher Bestandteile des Mittels umfassen.

Prinzipiell brauchbar sind beispielsweise Mineralöle, synthetische Öle sowie pflanzliche und tierische Öle, sowie niedermolekulare hydrophile Lösungsmittel wie Alkohole, Ether, Ketone und ähnliches.

Einerseits sind daher aprotische bzw. apolare Lösungs- bzw. Verdünnungsmittel zu nennen, wie Mineralölfraktionen von mittlerem bis hohem Siedepunkt, z.B. Kerosin und Dieselöl, ferner Kohlenteeröle, Kohlenwasserstoffe, Paraffinöle, z.B. $C_8$- bis $C_{30}$-Kohlenwasserstoffe der n- oder iso-Alkan-Reihe oder Gemische davon, gegebenenfalls hydrierte oder teilhydrierte Aromaten oder Alkylaromaten aus der Benzol- oder Naphthalin-Reihe, z.B. aromatische oder cycloaliphatische $C_7$- bis $C_{18}$-Kohlenwasserstoffverbindungen, aliphatische oder aromatische Carbonsäure- oder Dicarbonsäureester, Fette oder Öle pflanzlichen oder tierischen Ursprungs, wie Mono-, Di- und Triglyceride, in Reinform oder als Gemisch beispielsweise in Form öliger Naturstoffextrakte, z.B. Olivenöl, Sojaöl, Sonnenblumenöl, Castoröl, Sesamöl, Maisöl, Erdnussöl, Rapsöl, Leinsamenöl, Mandelöl, Rhizinusöl, Safloröl, sowie deren Raffinate, z.B. hydrierte oder teilhydrierte Produkte davon und/oder deren Ester, insbesondere Methyl- und Ethylester.

Beispiele für $C_8$- bis $C_{30}$-Kohlenwasserstoffe der n- oder iso-Alkan-Reihe sind n- und iso-Octan, -Decan, -Hexadecan, -Octadecan, -Eicosan, und vorzugsweise Kohlenwasserstoffgemische, wie Paraffinöl (das in technischer Qualität bis zu etwa 5% Aromaten enthalten kann) und ein $C_{18}$-$C_{24}$-Gemisch, das unter der Bezeichnung Spraytex-Öl im Handel von der Fa. Texaco erhältlich ist.

Zu den aromatischen oder cycloaliphatischen $C_7$- bis $C_{18}$-Kohlenwasserstoffverbindungen gehören insbesondere aromatische oder cycloaliphatische Lösungsmittel aus der Alkyl-Aromatenreihe. Diese Verbindungen können unhydriert, teilhydriert oder vollständig hydriert sein. Zu derartigen Lösungsmitteln gehören insbesondere Mono-, Di- oder Trialkylbenzole, Mono-, Di-, Trialkyl-substituierte Tetraline und/oder Mono-, Di-, Tri- oder Tetraalkyl-substituierte Naphthaline (Alkyl steht vorzugsweise für $C_1$-$C_6$-Alkyl). Beispiele derartiger Lösungsmittel sind Toluol, o-, m-, p-Xylol, Ethylbenzol, Isopropylbenzol, tert.-Butylbenzol und Gemische, wie die unter der Bezeichnung Shellsol und Solvesso vertriebenen Produkte der Fa. Exxon, z.B. Solvesso 100, 150 und 200.

Beispiele für geeignete Monocarbonsäureester sind Ölsäureester, insbesondere Methyloleat und Ethyloleat, Laurinsäureester, insbesondere 2-Ethylhexyllaurat, Octyllaurat und Isopropyllaurat, Isopropylmyristat, Palmitinsäureester, insbesondere 2-Ethylhexylpalmitat und Isopropylpalmitat, Stearinsäureester, insbesondere Stearinsäure-n-butylester und 2-Ethylhexansäure-2-ethylhexyl-ester.

Beispiele für geeignete Dicarbonsäureester sind Adipinsäureester, insbesondere Dimethyladipat, Di-n-butyladipat, Di-n-octyladipat, Di-iso-octyladipat, auch als Bis-(2-ethylhexyl)adipat bezeichnet, Di-n-nonyladidipat, Di-iso-nonyladidipat und Ditridecyladipat; Bernsteinsäureester, insbesondere Di-n-octylsuccinat und Di-iso-octylsuccinat, und Di-(iso-nonyl)cyclohexan-1,2-dicarboxylat.

Der Anteil der zuvor beschriebenen aprotischen Lösungs- bzw. Verdünnungsmitteln am Gesamtgewicht des Mittels beträgt in der Regel weniger als 80 Gew.-%, vorzugsweise weniger als 50 Gew.-% und insbesondere weniger als 30 Gew.-%.

Einige dieser aprotischen Lösungs- bzw Verdünnungsmittel können ebenfalls adjuvante, d.h. insbesondere wirkungsfördernde Eigenschaften, haben. Dies gilt insbesondere für besagte Mono- und Dicarbonsäureester. Unter diesem Aspekt können derartige Adjuvantien auch als Teil einer weiteren Formulierung (stand alone-Produkt) mit den erfindungsgemäßen Alkoholalkoxylaten bzw. diese enthaltenden Mitteln zu einem zweckmäßigen Zeitpunkt, in der Regel kurz vor der Applikation, vermischt werden.

Andererseits sind protische bzw. polare Lösungs- bzw. Verdünnungsmittel zu nennen, z.B. $C_2$-$C_8$-Monoalkohole wie Ethanol, Propanol, Isopropanol, Butanol, Isobutanol, tert-Butanol, Cyclohexanol und 2-Ethylhexanol, $C_3$-$C_8$-Ketone wie Diethylketon, t-Butylmethylketon, Cyclohexanon und 2-sec-Butylphenol, sowie aprotische Amine, wie N-Methyl- und N-Octylpyrrolidon.

Der Anteil der zuvor beschriebenen protischen bzw. polaren Lösungs- bzw. Verdünnungsmitteln am Gesamtgewicht des Mittels beträgt in der Regel weniger als 80 Gew.-%, vorzugsweise weniger als 50 Gew.-% und insbesondere weniger als 30 Gew.-%. Auch Antiabsetzmittel können insbesondere für Suspensionskonzentrate verwendet werden. Diese dienen vor allem zur rheologischen Stabilisierung. Insbesondere sind in diesem Zusammenhang mineralische Produkte, z.B. Bentonite, Talcite und Hektorite zu nennen.

Weitere gegebenenfalls brauchbare Zusätze sind z.B. unter Mineralsalzlösungen, welche zur Behebung von Ernährungs- und Spurenelementmängeln eingesetzt werden, nichtphytotoxischen Ölen und Ölkonzentraten, Antidriftreagenzien, Antischaummitteln, insbesondere solchen vom Silicon-Typ, beispielsweise das von der Firma Wacker vertriebene Silicon SL, und ähnlichem zu finden.

Gemäß einer bevorzugten Ausführungsform handelt es sich bei den erfindungsgemäßen Mitteln um Flüssigformulierungen.

Die Formulierungen können beispielsweise als emulgierbares Konzentrat (EC), Suspoemulsion (SE), Öl-in-Wasser-Emulsion (O/W), Wasser-in-Öl-Emulsion (W/O), wässriges Suspensionskonzentrat, Öl-Suspensionskonzentrat (OD), Mikroemulsion (ME) vorliegen.

Die Herstellung der Mittel kann in an sich bekannter Weise erfolgen. Dazu werden zumindest Teile der Komponenten zusammengegeben. Hierbei ist zu beachten, dass Produkte, insbesondere handelsübliche Produkte, verwendet werden können, deren Bestandteile zu unterschiedlichen Komponenten beitragen können. Beispielsweise kann ein bestimmtes Tensid in einem aprotischen Lösungsmittel gelöst sein, so dass dieses Produkt zu verschiedenen Komponenten beitragen kann. Ferner können unter Umständen auch geringe Anteile an weniger erwünschten Substanzen mit handelsüblichen Produkten eingebracht werden. Als Gemisch sind die zusammengegebenen Produkte dann in der Regel intensiv miteinander zu einem homogenen Gemisch zu vermengen und erforderlichenfalls - z.B. im Falle von Suspensionen, zu vermahlen.

Das Vermengen kann in an sich bekannter Weise erfolgen, z.B. durch Homogenisieren mit geeigneten Vorrichtungen wie KPG- oder Magnetrührern. Auch das Vermahlen ist ein an sich bekannter Vorgang. Als Mahlkörper kann man Glasmahlkörper oder andere mineralische oder metallische Mahlkörper, in der Regel in einer Größe von 0,1-30 mm und insbesondere von 0,6-2 mm verwenden. Man zerkleinert das Gemisch in der Regel solange, bis die gewünschte Partikelgröße erreicht ist.

Die Mittel werden vor Gebrauch in der Regel durch Verdünnen in üblicher Weise in eine zur Anwendung brauchbare Form überführt. Bevorzugt ist das Verdünnen mit Wasser oder auch aprotischen Lösungsmitteln, beispielsweise im Tankmixverfahren. Die Verwendung in Form einer Spritzbrühen-Zubereitung ist bevorzugt. Appliziert werden kann im Vorauflauf- oder Nachauflaufverfahren. Besondere Vorteile ergeben sich im Nachauflaufverfahren.

Die erfindungsgemäße Verwendung umfasst auch den Einsatz der erfindungsgemäßen Alkoxylate als "stand-alone"-Produkt. Dazu werden die Alkoxylate in geeigneter Weise hergerichtet, um kurz vor der Anwendung dem zu applizierenden Mittel zugesetzt zu werden. Für das Verhältnis von Alkoxylat zu Wirkstoff gelten die oben in Zusammenhang mit dem Mittel gemachten Aussagen. In diesem Sinne kann die erfindungsgemäße Kombination aus Wirkstoff und Adjuvans auch in Form eines Kits bereitgestellt werden. Ein solcher Kit beinhaltet zumindest zwei Behältnisse. Ein Behältnis umfasst wenigstens einen Wirkstoff zur Pflanzenbehandlung, gegebenenfalls als Mittel mit zweckmäßigen Hilfsstoffen formuliert. Ein weiteres Behältnis umfasst wenigstens ein Alkoholalkoxylat der Formel (I).

Vor allem bei der Sprühbehandlung ergeben sich besondere Vorteile. Für eine übliche Tankmix-Spritzbrühe werden die erfindungsgemäßen, bereits wenigstens einen alkoxyfierten verzweigten Alkohol enthaltenden Mittel - oder weitere Pflanzenbehandlungsmittel unter Zusatz wenigstens eines alkoxylierten verzweigten Alkohols als "stand-alone"-Produkt - so mit Wasser verdünnt, dass pro ha 0,01 bis 10, vorzugsweise 0,05 bis 5 und insbesondere 0,1 bis 1 kg wenigstens eines erfindungsgemäßen Alkoxylats appliziert werden.

Im Rahmen der vorliegenden Beschreibung sind Mengenangaben im Allgemeinen auf das Gesamtgewicht eines Mittels zu beziehen, sofern nicht anderes angeben ist. Der Ausdruck "im Wesentlichen" bezeichnet erfindungsgemäß in der Regel ein prozentuales Verhältnis von wenigstens 80 %, vorzugsweise von wenigstens 90 % und insbesondere von wenigstens 95 %.

Die Erfindung wird durch die nachfolgenden Beispiele näher erläutert:

Die gewichtsmittleren Molekuagewichte der erfindungsgemäßen Alkoxylate können mittels Gelpermeationschromatographie nach DIN 55672 bestimmt werden.

Vorschrift zur Bestimmung des Iso-Index eines Alkoholgemisches aus sekundären und/oder primären Alkoholen über $^1$H-NMR:

Ca. 20 mg Alkoholgemisch werden in 0,4 ml $CDCl_3$ gelöst und eine kleine Menge TMS zur Frequenzreferierung zugegeben. Danach wird die Lösung mit 0,2 ml TAI versetzt, in ein 5 mm NMR-Röhrchen gefüllt und im NMR-Spektrometer vermessen.

Messbedingungen:

Spektrometerfrequenz: 400 MHZ
Relaxationsdelay: 10 s
Pulswinkel: 30°
Aufgenommene Datenpunkte: 64 K
Scanzahl: 64
Transformierte Datenpunkte 64 K
Exponentialmultiplikation: 0,2 Hz

Nach Fouriertransformation, automatischer Phasen- und Basislinienkorrektur erfolgt eine manuelle Integration der Bereiche 5,4 bis 3,7 ppm (alle mit TAI veresterten sekundären bzw. primären Alkohole) und 2,4 bis 0,4 ppm (alle Methyl-, Methylen- und Methinprotonen). Dabei werden die Integralphasen nullter Ordnung so gewählt, dass Anfang und Ende der Integralkurven im Wesentlichen horizontal verlaufen. Die Signale < 1 ppm werden den Methylgruppen zugeordnet.

Herstellungsbeispiele

Referenzbeispiele 1 bis 24:

Herstellung der Alkoxylate (a) bis (x)

Referenzbeispiel 1: 1-Heptanol + 17 PO + 5 EO (a)

Man legte 116,2 g 1-Heptanol (entsprechend 1,0 Mol) zusammen mit 5,3 g 50% igem Kaliumhydroxid (Alkoxylierungskatalysator; entsprechend 0,2 Gew.-% bezogen auf den Gesamtansatz) in einem Rotationsverdampfer vor, man entwässerte und überführte das Reaktionsgemisch in einen Autoklaven.

Bei 130°C gaste man zunächst 986 g Propylenoxid (entsprechend 17,0 Mol) und anschließend 220,0 g Ethylenoxid (entsprechend 5,0 Mol) kontinuierlich ein.

Zur Vervollständigung der Umsetzung rührte man bei gleichzeitigem Abkühlen auf 80°C 30 Minuten nach.

Man erhielt 1336 g des Alkoholalkoxylats (a)

Referenzbeispiel 2: Dipropylenglykolmonomethylether + 20 PO + 1 EO (b)

Man legte 19,4 g Dipropylenglykolmonomethylether (entsprechend 0,12 Mol) zusammen mit 0,66 g 50% igem Kaliumhydroxid (Alkoxylierungskatalysator; entsprechend 0,2 Gew.-% bezogen auf den Gesamtansatz) in einem Autoklaven vor. Man entwässerte im Autoklaven.

Bei 130°C gaste man zunächst 139,2 g Propylenoxid (entsprechend 2,4 Mol) und anschließend 5,3 g Ethylenoxid (entsprechend 0,12 Mol) kontinuierlich ein.

Zur Vervollständigung der Umsetzung rührte man bei gleichzeitigem Abkühlen auf 80°C 30 Minuten nach.

Man erhielt 162,0 g des Alkoholalkoxylats (b)

Referenzbeispiel 3: Dipropylenglykolmonobutylether + 1 PO + 10 EO (c)

Man legte 47,5 g Dipropylenglykolmonobutylether (entsprechend 0,25 Mol) zusammen mit 0,69 g 50% igem Kaliumhydroxid (Alkoxylierungskatalysator; entsprechend 0,2 Gew.-% bezogen auf den Gesamtansatz) in einem Autoklaven vor. Man entwässerte im Autoklaven.

Bei 130°C gaste man zunächst 14,5 g Propylenoxid (entsprechend 0,25 Mol) und anschließend 110,0 g Ethylenoxid (entsprechend 2,5 Mol) kontinuierlich ein.

Zur Vervollständigung der Umsetzung rührte man bei gleichzeitigem Abkühlen auf 80°C 30 Minuten nach.

Man erhielt 158,0 g des Alkoholalkoxylats (c)

Referenzbeispiel 4: Dipropylenglykolmonopropylether + 20 PO + 10 EO (d)

Man legte 21,1 g Dipropylenglykolmonopropylether (entsprechend 0,12 Mol) zusammen mit 0,85 g 50% igem Kaliumhydroxid (Alkoxylierungskatalysator; entsprechend 0,2 Gew.-% bezogen auf den Gesamtansatz) in einem Autoklaven vor. Man entwässerte im Autoklaven.

Bei 130°C gaste man zunächst 139,2 g Propylenoxid (entsprechend 2,4 Mol) und anschließend 52,8 g Ethylenoxid (entsprechend 1,2 Mol) kontinuierlich ein.

Zur Vervollständigung der Umsetzung rührte man bei gleichzeitigem Abkühlen auf 80°C 30 Minuten nach.

Man erhielt 196,0 g des Alkoholalkoxylats (d)

Referenzbeispiel 5: Dipropylenglykolmonobutylether + 10,5 PO + 5,5 EO (e)

Man legte 28,5 g Dipropylenglykolmonobutylether (entsprechend 0,15 Mol) zusammen mit 0,62 g 50% igem Kaliumhydroxid (Alkoxylierungskatalysator; entsprechend 0,2 Gew.-% bezogen auf den Gesamtansatz) in einem Autoklaven vor. Man entwässerte im Autoklaven.

Bei 130°C gaste man zunächst 91,4 g Propylenoxid (entsprechend 1,6 Mol) und anschließend 36,3 g Ethylenoxid (entsprechend 0,83 Mol) kontinuierlich ein.

Zur Vervollständigung der Umsetzung rührte man bei gleichzeitigem Abkühlen auf 80°C 30 Minuten nach.

Man erhielt 145,0 g des Alkoholalkoxylats (e)

Referenzbeispiel 6: 1-Hexanol + 10,5 PO + 5,5 EO (f)

Man legte 20,4 g 1-Hexanol (entsprechend 0,2 Mol) zusammen mit 0,38 g Kalium-tert.-Butylat (Alkoxylierungskatalysator; entsprechend 0,2 Gew.-% bezogen auf den Gesamtansatz) in einem Autoklaven vor.

Bei 130°C gaste man zunächst 121,8 g Propylenoxid (entsprechend 2,1 Mol) und anschließend 48,4 g Ethylenoxid (entsprechend 1,1 Mol) kontinuierlich ein.

Zur Vervollständigung der Umsetzung rührte man bei gleichzeitigem Abkühlen auf 80°C 30 Minuten nach.

Man erhielt 189,0 g des Alkoholalkoxylats (f)

Referenzbeispiel 7: 1-Heptanol + 3 BO + 5 EO (g)

Man legte 116,2 g 1-Heptanol (ensprechend 1,0 Mol) zusammen mit 2,76 g Kalium-tert. Butylat (Alkoxylierungskatalysator; entsprechend 0,5 Gew.-% bezogen auf den Gesamtansatz) in einem Autoklaven vor.

Bei 130°C gaste man zunächst 216,0 g1,2-Butylenoxid (entsprechend 3,0 Mol) und anschliessend 220,0 g Ethylenoxid (entsprechend 5,0 Mol) ein.

Zur Vervollständigung der Umsetzung rührte man bei gleichzeitigem Abkühlen auf 80°C 30 Minuten nach.

Man erhielt 553,3 g des Alkoholalkoxylats (g)

Das mittels Gelpermeationschromatographie nach DIN 55672 bestimmte gewichtsmittlere Molekuagewicht beträgt ca. 550.

Referenzbeispiel 8: 1-Heptanol + 5 BO + 5 EO (h)

Man legte 116,2 g 1-Heptanol (ensprechend 1,0 Mol) zusammen mit 3,48 g Kalium-tert. Butylat (Alkoxylierungskatalysator; entsprechend 0,5 Gew.-% bezogen auf den Gesamtansatz) in einem Autoklaven vor.
Bei 130°C gaste man zunächst 360,0 g 1,2-Butylenoxid (entsprechend 5,0 Mol) und anschliessend 220,0 g Ethylenoxid (entsprechend 5,0 Mol) ein.
Zur Vervollständigung der Umsetzung rührte man bei gleichzeitigem Abkühlen auf 80°C 30 Minuten nach.

Man erhielt 727,3 g des Alkoholalkoxylats (h)

Das mittels Gelpermeationschromatographie nach DIN 55672 bestimmte gewichtsmittlere Molekuagewicht beträgt ca. 725.

Referenzbeispiel 9: 1-Heptanol + 7 BO + 5 EO (i)

Man legte 116,2 g 1-Heptanol (ensprechend 1,0 Mol) zusammen mit 4,2 g Kalium-tert. Butylat (Alkoxylierungskatalysator; entsprechend 0,5 Gew.-% bezogen auf den Gesamtansatz) in einem Autoklaven vor.
Bei 130°C gaste man zunächst 504,0 g 1,2-Butylenoxid (entsprechend 7,0 Mol) und anschliessend 220,0 g Ethylenoxid (entsprechend 5,0 Mol) ein.
Zur Vervollständigung der Umsetzung rührte man bei gleichzeitigem Abkühlen auf 80°C 30 Minuten nach.

Man erhielt 889,5 g des Alkoholalkoxylats (i)

Das mittels Gelpermeationschromatographie nach DIN 55672 bestimmte gewichtsmittlere Molekuagewicht beträgt ca. 840.

Referenzbeispiel 10: 1-Heptanol + 3 BO + 12 EO (j)

Man legte 116,2 g 1-Heptanol (ensprechend 1,0 Mol) zusammen mit 1,72 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 216,0 g 1,2-Butylenoxid entsprechend 3,0 Mol) und anschliessend 528,0 g Ethylenoxid (entsprechend 12,0 Mol) ein.
Zur Vervollständigung der Umsetzung rührte man bei gleichzeitigem Abkühlen auf 80°C 30 Minuten nach.

Man erhielt 844 g des Alkoholalkoxylats (j)

Das mittels Gelpermeationschromatographie nach DIN 55672 bestimmte gewichtsmittlere Molekuagewicht beträgt ca. 860.

Referenzbeispiel 11: 1-Heptanol + 9 BO + 5 EO (k)

Man legte 116,2 g 1-Heptanol (ensprechend 1,0 Mol) zusammen mit 1,97 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 648,0 g 1,2-Butylenoxid (entsprechend 9,0 Mol) und anschliessend 220,0 g Ethylenoxid (entsprechend 5,0 Mol) ein.
Zur Vervollständigung der Umsetzung rührte man bei gleichzeitigem Abkühlen auf 80°C 30 Minuten nach.

Man erhielt 970 g des Alkoholalkoxylats (k)

Das mittels Gelpermeationschromatographie nach DIN 55672 bestimmte gewichtsmittlere Molekuagewicht beträgt ca. 980.

Referenzbeispiel 12: 1-Heptanol + 9 BO + 12 EO (l)

Man legte 116,2 g 1-Heptanol (ensprechend 1,0 Mol) zusammen mit 2,58 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 648,0 g 1,2-Butylenoxid (entsprechend 9,0 Mol) und anschliessend 528,0 g Ethylenoxid (entsprechend 12,0 Mol) ein.

Zur Vervollständigung der Umsetzung rührte man bei gleichzeitigem Abkühlen auf 80°C 30 Minuten nach.

Man erhielt 1325 g des Alkoholalkoxylats (l)

Das mittels Gelpermeationschromatographie nach DIN 55672 bestimmte gewichtsmittlere Molekuagewicht beträgt ca. 1290.

Referenzbeispiel 13: 1-Heptanol + 12 EO + 9 BO (m)

Man legte 116,2 g 1-Heptanol (ensprechend 1,0 Mol) zusammen mit 1,97 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 528,0 g Ethylenoxid (entsprechend 12,0 Mol) und anschliessend 648,0 g 1,2-Butylenoxid (entsprechend 9,0 Mol) ein.
Zur Vervollständigung der Umsetzung rührte man bei gleichzeitigem Abkühlen auf 80°C 30 Minuten nach.

Man erhielt 1260 g des Alkoholalkoxylats (m)

Das mittels Gelpermeationschromatographie nach DIN 55672 bestimmte gewichtsmittlere Molekuagewicht beträgt ca. 1290.

Referenzbeispiel 14: 1-Heptanol + 3 BO + 12 EO + 1 i-BO (n)

Man legte 174,3 g 1-Heptanol (ensprechend 1,5 Mol) zusammen mit 2,58 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 324,0 g 1,2-Butylenoxid (entsprechend 3,0 Mol) und anschliessend 792,0 g Ethylenoxid (entsprechend 12,0 Mol) ein.
Zur Vervollständigung der Umsetzung rührte man bei gleichzeitigem Abkühlen auf 80°C 30 Minuten nach.
Man legte einen aliquoten Teil 403,0 g des erhaltenen Alkoholalkoxylats (entsprechend 0,5 Mol) zusammen mit 0,89 g Kalium-tert. Butylat (Alkoxylierungskatalysator; entsprechend 0,2 Gew.-% bezogen auf den Gesamtansatz) in einem Autoklaven vor.

Bei 140°C gaste man 43,2 g iso-Butylenoxid (entsprechend 0,6 Mol) ein.

Zur Vervollständigung der Umsetzung rührte man bei gleichzeitigem Abkühlen auf 80°C 30 Minuten nach.

Man erhielt 438 g des Alkoholalkoxylats (n)

Das mittels Gelpermeationschromatographie nach DIN 55672 bestimmte gewichtsmittlere Molekuagewicht beträgt ca. 880.

Referenzbeispiel 15: 1-Heptanol + 17 PO + 5 EO + 1 i-BO (o)

Man legte 116,2 g 1-Heptanol (ensprechend 1,0 Mol) zusammen mit 2,65 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 986,0 g 1,2-Propylenoxid (entsprechend 17,0 Mol) und anschliessend 220,0 g Ethylenoxid (entsprechend 5,0 Mol) ein.
Zur Vervollständigung der Umsetzung rührte man bei gleichzeitigem Abkühlen auf 80°C 30 Minuten nach.
Man legte einen aliquoten Teil 462,8 g des erhaltenen Alkoholalkoxylats (entsprechend 0,4 Mol) zusammen mit 1,0 g Kalium-tert. Butylat (Alkoxylierungskatalysator; entsprechend 0,2 Gew.-% bezogen auf den Gesamtansatz) in einem Autoklaven vor.
Bei 140°C gaste man 34,6 g iso-Butylenoxid (entsprechend 0,48 Mol) ein.
Zur Vervollständigung der Umsetzung rührte man bei gleichzeitigem Abkühlen auf 80°C 30 Minuten nach.

Man erhielt 487 g des Alkoholalkoxylats (o)

Das mittels Gelpermeationschromatographie nach DIN 55672 bestimmte gewichtsmittlere Molekuagewicht beträgt ca. 1230.

Referenzbeispiel 16: 1-Heptanol + 3 BO + 12 EO + DMS (p)

Man legte 174,3 g 1-Heptanol (ensprechend 1,5 Mol) zusammen mit 2,58 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 324,0 g 1,2-Butylenoxid (entsprechend 3,0 Mol) und anschliessend 792,0 g Ethylenoxid (entsprechend 12,0 Mol) ein.
Zur Vervollständigung der Umsetzung rührte man bei gleichzeitigem Abkühlen auf 80°C 30 Minuten nach.
Man legte einen aliquoten Teil, 322,4 g (entsprechend 0,4 Mol) des erhaltenen Alkoxylats in einem Vierhalskolben vor tropfte unter Kühlung 163,2 g 50% ige Natronlauge (entsprechend 2,04 Mol) unter Kühlung zu. Danach wurden bis max. 40°C 65,6 g Dimethylsulfat (entsprechend 0,52 Mol) in das Reaktionsgemisch zudosiert.

Man erhielt 334,1 g des modifizierten Alkoholalkoxylats (p)

Das mittels Gelpermeationschromatographie nach DIN 55672 bestimmte gewichtsmittlere Molekuagewicht beträgt ca. 880.

Referenzbeispiel 17: 1-Heptanol + 17 PO + 5 EO + DMS (q)

Man legte 116,2 g 1-Heptanol (ensprechend 1,0 Mol) zusammen mit 2,65 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 986,0 g 1,2-Propylenoxid (entsprechend 17,0 Mol) und anschliessend 220,0 g Ethylenoxid (entsprechend 5,0 Mol) ein.
Zur Vervollständigung der Umsetzung rührte man bei gleichzeitigem Abkühlen auf 80°C 30 Minuten nach.
Man legte einen aliquoten Teil, 462,8 g (entsprechend 0,4 Mol) des erhaltenen Alkoxylats in einem Vierhalskolben vor tropfte unter Kühlung 163,2 g 50% ige Natronlauge (entsprechend 2,04 Mol) unter Kühlung zu. Danach wurden bis max. 40°C 65,6 g Dimethylsulfat (entsprechend 0,52 Mol) in das Reaktionsgemisch zudosiert.

Man erhielt 451 g des modifizierten Alkoholalkoxylats (q)

Das mittels Gelpermeationschromatographie nach DIN 55672 bestimmte gewichtsmittlere Molekuagewicht beträgt ca. 1340.

Referenzbeispiel 18: Dipropylenglykolmonobutylether + 3 BO + 12 EO (r)

Man legte 190,0 g Dipropylenglykolmonobutylether (ensprechend 1,0 Mol) zusammen mit 3,72 g 50%ige wässrige Kaliumhydroxidlsg. (Alkoxylierungs-katalysator; entsprechend 0,2 Gew.-% bezogen auf den Gesamtansatz) in einem Autoklaven vor.
Bei 130°C gaste man zunächst 216,0 g 1,2-Butylenoxid (entsprechend 3,0 Mol) und anschliessend 528,0 g Ethylenoxid (entsprechend 12,0 Mol) ein.
Zur Vervollständigung der Umsetzung rührte man bei gleichzeitigem Abkühlen auf 80°C 30 Minuten nach.

Man erhielt 930 g des Alkoholalkoxylats (r)

Das mittels Gelpermeationschromatographie nach DIN 55672 bestimmte gewichtsmittlere Molekuagewicht beträgt ca. 930.

Referenzbeispiel 19: Dipropylenglykolmonobutylether + 1 BO + 12 EO (s)

Man legte 190,0 g Dipropylenglykolmonobutylether (ensprechend 1,0 Mol) zusammen mit 3,16 g 50%ige wässrige Kaliumhydroxidlsg. (Alkoxylierungs-katalysator; entsprechend 0,2 Gew.-% bezogen auf den Gesamtansatz) in einem Autoklaven vor.
Bei 130°C gaste man zunächst 72,0 g 1,2-Butylenoxid (entsprechend 1,0 Mol) und anschliessend 528,0 g Ethylenoxid (entsprechend 12,0 Mol) ein.
Zur Vervollständigung der Umsetzung rührte man bei gleichzeitigem Abkühlen auf 80°C 30 Minuten nach.

Man erhielt 789 g des Alkoholalkoxylats (s)

Das mittels Gelpermeationschromatographie nach DIN 55672 bestimmte gewichtsmittlere Molekuagewicht

beträgt ca. 790.

Referenzbeispiel 20: Dipropylenglykolmonobutylether + 1 PeO + 12 EO (t)

Man legte 190,0 g Dipropylenglykolmonobutylether (ensprechend 1,0 Mol) zusammen mit 1,61 g 50%ige wässrige Kaliumhydroxidlsg. (Alkoxylierungs-katalysator; entsprechend 0,2 Gew.-% bezogen auf den Gesamtansatz) in einem Autoklaven vor.
Bei 150°C gaste man zunächst 86,0 g 1,2-Pentenoxid (entsprechend 1,0 Mol) und anschliessend bei 130°C 528,0 g Ethylenoxid (entsprechend 12,0 Mol) ein.
Zur Vervollständigung der Umsetzung rührte man bei gleichzeitigem Abkühlen auf 80°C 30 Minuten nach.

Man erhielt 789 g des Alkoholalkoxylats (t)

Das mittels Gelpermeationschromatographie nach DIN 55672 bestimmte gewichtsmittlere Molekuagewicht beträgt ca. 804.

Referenzbeispiel 21: Dipropylenglykolmonobutylether + 1 DeO + 12 EO (u)

Man legte 190,0 g Dipropylenglykolmonobutylether (ensprechend 1,0 Mol) zusammen mit 1,75 g 50%ige wässrige Kaliumhydroxidlsg. (Alkoxylierungs-katalysator; entsprechend 0,2 Gew.-% bezogen auf den Gesamtansatz) in einem Autoklaven vor.
Bei 150°C gaste man zunächst 156,0 g 1,2-Decenoxid (entsprechend 1,0 Mol) und anschliessend bei 130°C 528,0 g Ethylenoxid (entsprechend 12,0 Mol) ein.
Zur Vervollständigung der Umsetzung rührte man bei gleichzeitigem Abkühlen auf 80°C 30 Minuten nach.

Man erhielt 866 g des Alkoholalkoxylats (u)

Das mittels Gelpermeationschromatographie nach DIN 55672 bestimmte gewichtsmittlere Molekuagewicht beträgt ca. 874.

Referenzbeispiel 22: Dipropylenglykolmonobutylether + 12 EO + 1 PeO (v)

Man legte 190,0 g Dipropylenglykolmonobutylether (ensprechend 1,0 Mol) zusammen mit 1,61 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 528,0 g Ethylenoxid (entsprechend 12,0 Mol) und anschliessend bei 150°C 86,0 g 1,2-Pentenoxid (entsprechend 1,0 Mol) ein.
Zur Vervollständigung der Umsetzung rührte man bei gleichzeitigem Abkühlen auf 80°C 30 Minuten nach.

Man erhielt 815 g des Alkoholalkoxylats (v)

Das mittels Gelpermeationschromatographie nach DIN 55672 bestimmte gewichtsmittlere Molekuagewicht beträgt ca. 805.

Referenzbeispiel 23: Dipropylenglykolmonobutylether + 12 EO + 1 DeO (w)

Man legte 190,0 g Dipropylenglykolmonobutylether (ensprechend 1,0 Mol) zusammen mit 1,75 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 528,0 g Ethylenoxid (entsprechend 12,0 Mol) und anschliessend bei 150°C 156,0 g 1,2-Decenoxid (entsprechend 1,0 Mol) ein.
Zur Vervollständigung der Umsetzung rührte man bei gleichzeitigem Abkühlen auf 80°C 30 Minuten nach.

Man erhielt 895 g des Alkoholalkoxylats (w)

Das mittels Gelpermeationschromatographie nach DIN 55672 bestimmte gewichtsmittlere Molekuagewicht beträgt. 874.

Referenzbeispiel 24: Pentanolgemisch + 7 BO + 12 EO (x)

Man legte 87,2 g Pentanolgemisch (ensprechend 1,0 Mol) zusammen mit 2,23 g Kalium-tert. Butylat (Alkoxylierungskatalysator; entsprechend 0,2 Gew.-% bezogen auf den Gesamtansatz) in einem Autoklaven vor.
Bei 130°C gaste man zunächst 504,0 g 1,2-Butylenoxid (entsprechend 7,0 Mol) und anschliessend 528,0 g Ethylenoxid (entsprechend 12,0 Mol) ein.
Zur Vervollständigung der Umsetzung rührte man bei gleichzeitigem Abkühlen auf 80°C 30 Minuten nach.

Man erhielt 1116 g des Alkoholalkoxylats (x)

Das mittels Gelpermeationschromatographie nach DIN 55672 bestimmte gewichtsmittlere Molekuagewicht beträgt ca. 1120.

Beispiel 1: Fungizide Wirksamkeit

125 g/l Epoxiconazol wurden in einer Rührwerkskugelmühle (Dynomühle) zusammen mit jeweils 20 g/l Dispergiermittel (Atlas G 5000[1], Synperonic A[1]) sowie 50 g/l Propylenglykol im wässrigen Medium gemahlen bis eine Teilchengröße von 80 % < 2 μm erreicht wurde. Zu der Mischung kamen 3 g/l pro Liter Antischaummittel z.B. Rhodorsil 426[2], 3 g/l Verdicker, z.B. Rhodopol 23[2] sowie ein Biozid z.b. Acticide MBS[3]. Zu dieser Mischung wurde das jeweilige Adjuvans in wässriger Lösung oder in einem Lösungsmittel z.B. Solvesso dazugerührt, so dass die Endkonzentration der Formulierung aus 62.5 g/l Epoxiconazol und 125 g/l Adjuvans besteht.
[1] Uniquema/Croda
[2] Rhodia
[3] Thor Chemie
Biologische Prüfung (kurative Bekämpfung von Weizenbraunrost):

Blätter von in Töpfen gewachsenen Weizenkeimlingen der Sorte "Kanzler" wurden im Zweiblattstadium mit Sporen des Weizenbraunrostes "Puccinia recondita" bestäubt und 2 Tage im Gewächshaus bei hoher Luftfeuchtigkeit inkubiert. Dann wurden die Pflanzen in vollautomatischen Spritzkabinen mit den Formulierungen besprüht, welche die unten angegebenen Wirkstoffe und Adjuvantien enthielten. Die Spritzbrühen enthielten 50 ppm Epoxiconazol und 100 ppm Adjuvans. Das Verhältnis Wirkstoff zu Adjuvans betrug demnach 1:2. Nach dem Eintrocknen der Spritzbeläge wurden die Pflanzen ins Gewächshaus zurückgestellt und bei Temperaturen zwischen 20 und 24 °C sowie 60 bis 90 % relativer Luftfeuchtigkeit kultiviert. Nach 10 Tagen wurde das Ausmaß des Braunrostbefalls visuell als prozentualen Befall der gesamten Blattfläche ermittelt. Für jedes Versuchsglied wurden 3 Töpfe ausgewertet.

| Adjuvans [100 ppm] | Wirkstoff [50 ppm] | % Befall |
|---|---|---|
| (a) | Epoxiconazol | 0 |
| (b) | Epoxiconazol | 1 |
| (c) | Epoxiconazol | 4 |
| (d) | Epoxiconazol | 0 |
| (e) | Epoxiconazol | 0 |
| (f) | Epoxiconazol | 0 |
| (g) | Epoxiconazol | 3 |
| (h) | Epoxiconazol | 2 |
| (i) | Epoxiconazol | 1 |
| (j) | Epoxiconazol | 3 |
| (k) | Epoxiconazol | 5 |
| (l) | Epoxiconazol | 0 |
| (m) | Epoxiconazol | 4 |
| (n) | Epoxiconazol | 2 |

(fortgesetzt)

| Adjuvans [100 ppm] | Wirkstoff [50 ppm] | % Befall |
|---|---|---|
| (o) | Epoxiconazol | 0 |
| (p) | Epoxiconazol | 2 |
| (q) | Epoxiconazol | 0 |
| (r) | Epoxiconazol | 3 |
| (s) | Epoxiconazol | 7 |
| (t) | Epoxiconazol | 5 |
| (u) | Epoxiconazol | 3 |
| (v) | Epoxiconazol | 3 |
| (w) | Epoxiconazol | 1 |
| (x) | Epoxiconazol | 0 |
| LeoFAT | Epoxiconazol | 7 |
| - | Epoxiconazol | 40 |
| - | - | 90 |

| | |
|---|---|
| (a) | 1 Heptanol + 17 PO + 5 EO |
| (b) | Dipropylenglykolmonomethylether + 20 PO + 1 EO |
| (c) | Dipropylenglykolmonobutylether + 1 PO + 10 EO |
| (d) | Dipropylenglykolmonopropylether + 20 PO + 10 EO |
| (e) | Dipropylenglykolmonobutylether + 10,5 PO + 5,5 EO |
| (f) | 1-Hexanol + 10,5 PO + 5,5 EO |
| (g) | 1-Heptanol + 3 BO + 5 EO |
| (h) | 1-Heptanol + 5 BO + 5 EO |
| (i) | 1-Heptanol + 7 BO + 5 EO |
| (j) | 1-Heptanol + 3 BO + 12 EO |
| (k) | 1-Heptanol + 9 BO + 5 EO |
| (l) | 1-Heptanol + 9 BO + 12 EO |
| (m) | 1-Heptanol + 12 EO + 9 BO |
| (n) | 1-Heptanol + 3 BO + 12 EO + 1 i-BO |
| (o) | 1-Heptanol + 17 PO + 5 EO + 1 i-BO |
| (p) | 1-Heptanol + 3 BO + 12 EO + DMS |
| (q) | 1-Heptanol + 17 PO + 5 EO + DMS |
| (r) | Dipropylenglykolmonobutylether + 3 BO + 12 EO |
| (s) | Dipropylenglykolmonobutylether + 1 BO + 12 EO |
| (t) | Dipropylenglykolmonobutylether + 1 PeO + 12 EO |
| (u) | Dipropylenglykolmonobutylether + 1 DeO + 12 EO |
| (v) | Dipropylenglykolmonobutylether + 12 EO + 1 PeO |
| (w) | Dipropylenglykolmonobutylether + 12 EO + 1 PeO |

(fortgesetzt)

| | |
|---|---|
| (x) | Pentanolgemisch + 7 BO + 12 EO |
| LeoFAT | Fettsäuremethylester-ethoxyliert |

**Patentansprüche**

1. Mittel, umfassend

(a) wenigstens einen Wirkstoff zur Pflanzenbehandlung; und
(b) wenigstens einen alkoxylierten Alkohol der Formel (IIa)

$$R\text{-}O\text{-}(C_mH_{2m}O)_q\text{-}(C_nH_{20}O)_x\text{-}(C_2H_4O)_y\text{-}Z \qquad \text{(IIa)},$$

worin

R für $C_1$-$C_7$-Alkyl steht;
m für 2 oder 3 steht;
q für 0, 1, 2 oder 3 steht;
n für eine ganze Zahl von 3 bis 16 steht;
x für einen Wert von 1 bis 100 steht;
y y größer als 0 ist und für einen Wert bis 100 steht;
x+y einem Wert von 10 bis 30 entspricht; und
Z für Wasserstoff oder $C_1$-$C_3$-Alkyl steht,

wobei das Gewichtsverhältnis von Komponente (b) zu Komponente (a) größer als 1 ist.

2. Mittel nach Anspruch 1, **dadurch gekennzeichnet, dass** der alkoxylierte Alkohol ein alkoxylierter Alkohol der Formel (III)

$$R\text{-}O\text{-}(C_mH_{2m}O)_q\text{-}(C_3H_6O)_x\text{-}(C_2H_4O)_y\text{-}Z \qquad \text{(III)},$$

ist, worin R, m, q, Z wie in Anspruch 1 definiert sind, y für einen Wert von 1 bis 100 steht und der Wert von x größer als 5 ist.

3. Mittel nach Anspruch 1, **dadurch gekennzeichnet, dass** der alkoxylierte Alkohol ein alkoxylierter Alkohol der Formel (IV)

$$R\text{-}O\text{-}(C_mH_{2m}O)_q\text{-}(C_4H_8O)_x\text{-}(C_2H_4O)_y\text{-}Z \qquad \text{(IV)},$$

ist, worin R, m, q, Z wie in Anspruch 1 definiert sind, y für einen Wert von 1 bis 100 steht und der Wert von x größer als 2 ist.

4. Mittel nach Anspruch 1, **dadurch gekennzeichnet, dass** der alkoxylierte Alkohol ein alkoxylierter Alkohol der Formel (V)

$$R\text{-}O\text{-}(C_mH_{2m}O)_q\text{-}(C_5H_{100})_x\text{-}(C_2H_4O)_y\text{-}Z \qquad \text{(V)},$$

ist, worin R, m, q, Z wie in Anspruch 1 definiert sind, y für einen Wert von 1 bis 100 steht und der Wert von x größer als 2 ist.

5. Mittel nach einem der Ansprüche 1 bis 4, **dadurch gekennzeichnet, dass** die Summe aus x und y einem Wert von 10 bis 25 entspricht.

6. Mittel nach Anspruch 3, **dadurch gekennzeichnet, dass** der Rest R und die Gruppe -$(C_4H_8O)_x$- zusammengenommen mindestens 15 Kohlenstoffatome aufweisen.

7. Mittel nach Anspruch 4, **dadurch gekennzeichnet, dass** der Rest R und die Gruppe $-(C_5H_{10}O)_x-$ zusammengenommen mindestens 15 Kohlenstoffatome aufweisen.

8. Mittel nach einem der Ansprüche 1 bis 7, **dadurch gekennzeichnet, dass** R für $C_5$-$C_7$-Alkyl steht.

9. Mittel nach einem der Ansprüche 1 bis 8, **dadurch gekennzeichnet, dass** q Null ist.

10. Mittel nach einem der Ansprüche 1 bis 8, **dadurch gekennzeichnet, dass** q 1, 2 oder 3 ist.

11. Mittel nach einem der Ansprüche 1 bis 10, **dadurch gekennzeichnet, dass** m = 3 ist.

12. Mittel nach einem der Ansprüche 1 bis 11, **dadurch gekennzeichnet, dass** der Anteil der Komponente (b) am Gesamtgewicht des Mittels mehr als 10 Gew.-% beträgt.

**Claims**

1. A composition, comprising

(a) at least one active ingredient for the treatment of plants; and
(b) at least one alkoxylated alcohol of the formula (IIa)

$$R\text{-}O\text{-} (C_mH_{2m}O)_q\text{-} (C_nH_{2n}O)_x\text{-} (C_2H_4O)_y\text{-}Z \qquad (IIa)$$

in which

R represents $C_1$-$C_7$-alkyl;
m represents 2 or 3;
q represents 0, 1, 2 or 3;
n represents an integer from 3 to 16;
x represents a value of from 1 to 100;
y y is greater than 0 and represents a value of up to 100;
x+y corresponds to a value of from 10 to 30; and
Z represents hydrogen or $C_1$-$C_3$-alkyl,

where the weight ratio of component (b) to component (a) is greater than 1.

2. The composition according to claim 1, wherein the alkoxylated alcohol is an alkoxylated alcohol of the formula (III)

$$R\text{-}O\text{-} (C_mH_{2m}O)_q\text{-} (C_3H_6O)_x\text{-} (C_2H_4O)_y\text{-}Z \qquad (III)$$

in which R, m, q, Z are as defined in claim 1, y represents a value of from 1 to 100 and the value of x is greater than 5.

3. The composition according to claim 1, wherein the alkoxylated alcohol is an alkoxylated alcohol of the formula (IV)

$$R\text{-}O\text{-}(C_mH_{2m}O)q\text{-}(C_4H_8O)_x\text{-} (C_2H_4O)_y\text{-}z \qquad (IV),$$

in which R, m, q, Z are as defined in claim 1, y represents a value of from 1 to 100 and the value of x is greater than 2.

4. The composition according to claim 1, wherein the alkoxylated alcohol is an alkoxylated alcohol of the formula (V)

$$R\text{-}O\text{-}(C_mH_{2m}O)_q\text{-}(C_5H_{10}O)_x\text{-}(C_2H_4O)_y\text{-}Z \qquad (V),$$

in which R, m, q, Z are as defined in claim 1, y represents a value of from 1 to 100 and the value of x is greater than 2.

5. The composition according to any of claims 1 to 4, wherein the sum of x and y corresponds to a value of from 10 to 25.

6. The composition according to claim 3, wherein the radical R and the group $-(C_4H_8O)_x-$ together have at least 15

carbon atoms.

**7.** The composition according to claim 4, wherein the radical R and the group - $(C_5H_{10}O)_x$- together have at least 15 carbon atoms.

**8.** The composition according to any of claims 1 to 7, wherein R is $C_5$-$C_7$-alkyl.

**9.** The composition according to any of claims 1 to 8, wherein q is zero.

**10.** The composition according to any of claims 1 to 8, wherein q is 1, 2 or 3.

**11.** The composition according to any of claims 1 to 10, wherein m = 3.

**12.** The composition according to any of claims 1 to 11, wherein the proportion of component (b) of the total weight of the composition is more than 10% by weight.

**Revendications**

**1.** Agent, comprenant

(a) au moins une substance active pour le traitement de plantes ; et
(b) au moins un alcool alcoxylé de formule (IIa)

$$R\text{-}O\text{-}(C_mH_{2m}O)_q\text{-}(C_nH_{2n}O)_x\text{-}(C_2H_4O)_y\text{-}Z \qquad (IIa),$$

dans laquelle

R représente $C_1$-$C_7$-alkyle ;
m vaut 2 ou 3 ;
q vaut 0, 1, 2 ou 3 ;
n représente un nombre entier de 3 à 16 ;
x représente une valeur de 1 à 100 ;
y y est supérieur à 0 et représente une valeur jusqu'à 100 ;
x+y correspond à une valeur de 10 à 30 ; et
Z représente hydrogène ou $C_1$-$C_3$-alkyle,

le rapport pondéral du composé (b) au composant (a) étant supérieur à 1.

**2.** Agent selon la revendication 1, **caractérisé en ce que** l'alcool alcoxylé est un alcool alcoxylé de formule (III)

$$R\text{-}O\text{-}(C_mH_{2m}O)_q\text{-}(C_3H_{60})_x\text{-}(C_2H_4O)\ _y\text{-}Z \qquad (III)\ ,$$

dans laquelle R, m, q, Z sont définis comme dans la revendication 1, y représente une valeur de 1 à 100 et la valeur de x est supérieure à 5.

**3.** Agent selon la revendication 1, **caractérisé en ce que** l'alcool alcoxylé est un alcool alcoxylé de formule (IV)

$$R\text{-}O\text{-}(C_mH_{2m}O)q\text{-}(C_4H_8O)_x\text{-}(C_2H_4O)\ _y\text{-}Z \qquad (IV),$$

dans laquelle R, m, q, Z sont définis comme dans la revendication 1, y représente une valeur de 1 à 100 et la valeur de x est supérieure à 2.

**4.** Agent selon la revendication 1, **caractérisé en ce que** l'alcool alcoxylé est un alcool alcoxylé de formule (V)

$$R\text{-}O\text{-}(C_mH_{2m}O)_q\text{-}(C_5H_{10}O)_x\text{-}(C_2H_4O)_y\text{-}Z \qquad (V),$$

dans laquelle R, m, q, Z sont définis comme dans la revendication 1, y représente une valeur de 1 à 100 et la valeur

de x est supérieure à 2.

**5.** Agent selon l'une quelconque des revendications 1 à 4, **caractérisé en ce que** la somme de x et y correspond à une valeur de 10 à 25.

**6.** Agent selon la revendication 3, **caractérisé en ce que** le radical R et le groupe $-(C_4H_8O)_x-$, pris ensemble, présentent au moins 15 atomes de carbone.

**7.** Agent selon la revendication 4, **caractérisé en ce que** le radical R et le groupe $-(C_5H_{10}O)_x-$, pris ensemble, présentent au moins 15 atomes de carbone.

**8.** Agent selon l'une quelconque des revendications 1 à 7, **caractérisé en ce que** R représente $C_5$-$C_7$-alkyle.

**9.** Agent selon l'une quelconque des revendications 1 à 8, **caractérisé en ce que** q vaut zéro.

**10.** Agent selon l'une quelconque des revendications 1 à 8, **caractérisé en ce que** q vaut 1, 2 ou 3.

**11.** Agent selon l'une quelconque des revendications 1 à 10, **caractérisé en ce que** m = 3.

**12.** Agent selon l'une quelconque des revendications 1 à 11, **caractérisé en ce que** la proportion du composant (b) par rapport au poids total de l'agent est supérieure à 10% en poids.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0042847 A
- WO 03090531 A
- WO 2005015998 A
- WO 0035278 A
- WO 9903345 A
- WO 2005084435 A
- US 6071857 A
- JP 02049701 A
- EP 0261492 A
- JP 06313191 B
- GB 2109403 A
- JP 2001163813 A
- JP 9031004 A
- WO 2006070816 A
- US 6025318 A
- EP 0302487 A
- EP 0161537 A
- EP 897904 A
- WO 03075663 A
- WO 9846608 A
- WO 9941255 A
- WO 03004465 A
- WO 9626202 A
- WO 9741116 A
- WO 9741117 A
- WO 9741118 A
- EP 462456 A